(19) Europäisches Patentamt
European Patent Office
Office européen des brevets

(11) Publication number: **0 665 020 A2**

# (12) EUROPEAN PATENT APPLICATION

(21) Application number: **95101165.9**

(51) Int. Cl.[6]: **A61K 47/48**

(22) Date of filing: **27.01.95**

(30) Priority: **27.01.94 US 187337**

(43) Date of publication of application: **02.08.95 Bulletin 95/31**

(84) Designated Contracting States: **AT BE CH DE DK ES FR GB GR IE IT LI LU MC NL PT SE**

(71) Applicant: **BRISTOL-MYERS SQUIBB COMPANY**
**P.O. Box 4000**
**Princeton, NJ 08543-4000 (US)**

(72) Inventor: **Thottathil, John K.**
**31 Ellsworth Drive**
**Robbinsville, NJ (US)**
Inventor: **Pendri, Yadagiri**
**108 Winston Dr.**
**Matawan, NJ (US)**
Inventor: **Gadamasetti, Kumar G.**
**62 Reed Drive South**
**Princeton Junction, NJ (US)**
Inventor: **Li, Wen-Sen**
**3 Holly Hill Road**
**Marlboro, NJ (US)**
Inventor: **Mueller, Richard H.**
**66 Lindbergh Road**
**Ringoes, NJ (US)**

(74) Representative: **VOSSIUS & PARTNER**
**Siebertstrasse 4**
**D-81675 München (DE)**

## (54) Method for preparing thioether conjugates.

(57) A method is provided for preparing thioether conjugates useful in treating neoplastic diseases and preferably formed of the BR96 immunoconjugate synthesized from a heterobifunctional linker of doxorubicin, wherein a purified and reduced form of BR96 MAb is conjugated with the 6-maleimidocaproyl hydrazone of doxorubicin and the resulting crude conjugate is purified.

EP 0 665 020 A2

Bifunctional compounds which link cytotoxic reagents to antibodies are known. These compounds have been particularly useful in the formation of immunoconjugates directed against tumor associated antigens. Such immunoconjugates allow the selective delivery of toxic drugs to tumor cells. (See e.g., Hermentin and Seiler, "Investigations With Monoclonal Antibody Drug Conjugates," Behring Insti. Mitl. 82:197-215 (1988); Gallego et al., "Preparation of Four Daunomycin-Monoclonal Antibody 79IT/36 Conjugates With Anti-Tumor Activity". Int. J. Cancer 33:737-744 (1984); Arnon et al, "In Vitro and In Vivo Efficacy of Conjugates of Daunomycin With Anti-Tumor Antibodies," Immunological Rev. 62:5-27 (1982).

Greenfield et al have recently described the formation of acid-sensitive immunoconjugates containing the acylhydrazine compound, 3-(2-pyridyldithio)propionyl hydrazide conjugated via an acylhydrazone bond to the 13-keto position of an anthracycline molecule, and conjugation of this anthracycline derivative to an antibody molecule (Greenfield et al, European Patent Publication EP 0 328 147, published August 16, 1989, which corresponds to pending U.S. Serial No. 07/270,509, filed November 16, 1988, and U.S. Serial No. 07/155,181, filed February 11, 1988, now abandoned). This latter reference also discloses specific thioether-containing linkers and conjugates, including hydrazone thioether containing immunoconjugates.

Kaneko et al (U.S. Serial No. 07/522,996, filed May 14, 1990, which is equivalent to European Patent Publication, EP A 0 457 250, published November 21, 1991) have also described the formation of conjugates containing anthracycline antibiotics attached to a bifunctional linker by an acylhydrazone bond at the C-13 position of an anthracycline molecule. In their invention the linkers contain a reactive pyridinyldithio- or an ortho-nitrophenyldithio- group, by which the linker reacts with a suitable group attached to a cell reactive ligand, to form the complete conjugate.

U.S. Patent Application Serial No. 824,951, filed January 23, 1992, by D. Willner et al (which is incorporated herein by reference) discloses methods for preparing therapeutically active conjugates employing an acid-sensitive linkage for linking a therapeutically active drug molecule to a ligand capable of recognizing a selected target cell population.

The conjugate formed by Willner et al has the general structure of Formula (I):

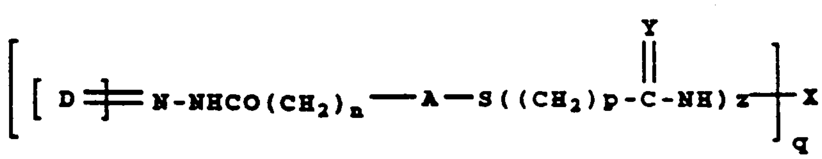

in which

D is a drug moiety;
n is 1 to 10;
p is 1 to 6;
Y is O or $NH_2^+Cl^-$;
z is 0 or 1;
q is about 1 to about 10;
x is a ligand; and,
A is Michael Addition Adduct moiety.

In a preferred embodiment, the drug, an anthracycline, preferably adriamycin (doxorubicin), is bound to the linker portion of the conjugate through an acylhydrazone bond at the 13-keto position of the anthracycline compound. The ligand which is an antibody reduced, preferably a chimeric BR96 antibody, then is bound, through the linker, preferably a maleimido group, to the anthracycline compound. In an especially preferred embodiment, this linkage occurs through a reduced disulfide group (i.e. a free sulfhydryl group (-SH)) on the antibody).

In the preferred method for preparing the above conjugates, as disclosed in the above U.S. application of Willner et al, a sulfhydryl group on the ligand $MAb-(SH)_8$, reacts directly with the Michael Addition Receptor of intermediate of Formula (IIb)

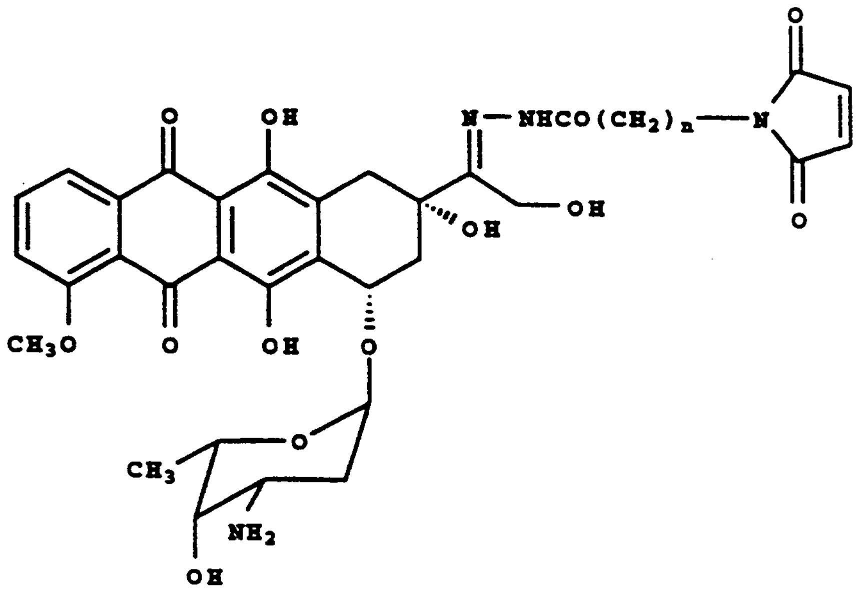

to form the final conjugate (Ia)

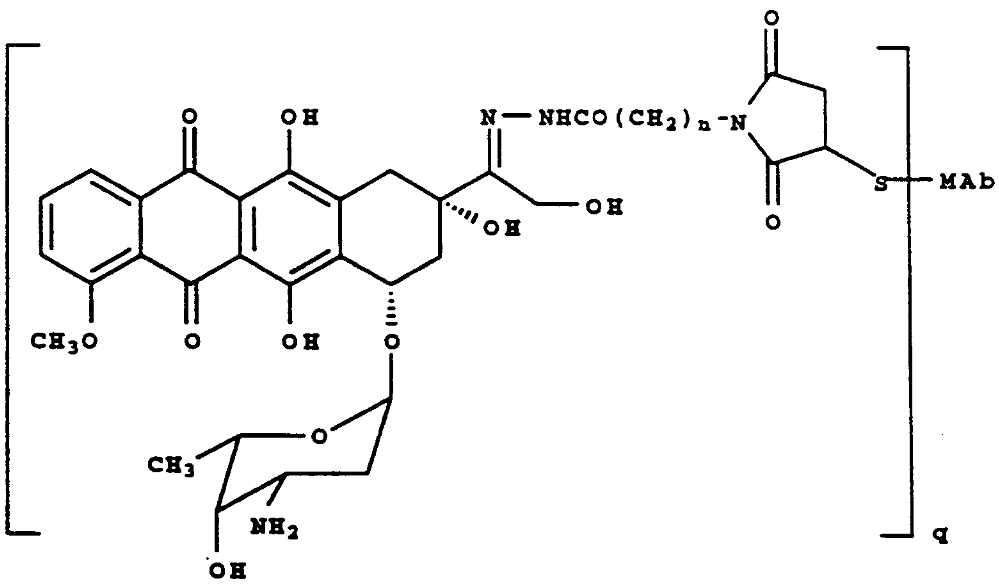

Using this process, generally between about one and about ten drug molecules may be linked to each ligand. Thus, in Formula (Ia), q may be from about 1 to about 10.

As described in the U.S. application of Willner et al, the intermediate Michael Addition Receptor containing hydrazone drug derivative of formula (IIb) may be prepared, depending on the Michael Addition Receptor moiety used, by reaction of the drug (or derivatized drug) with a hydrazide containing a Michael Addition Receptor in the general manner described in Method A:

Method A

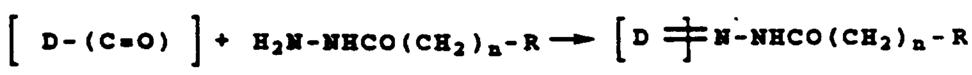

(IIc)

where D is doxorubicin, and R is a maleimide moiety.

The sulfhydryl containing ligand (III) (MAb-$(SH)_8$) may be produced, for example, by reduction of a disulfide bond in a native molecule II

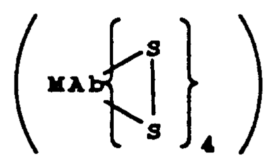

using a reducing agent useful for such purposes, for example, dithiothreitol ("DTT").

After the reaction of the conjugation is complete, the conjugate may be isolated and purified using known dialysis, chromatographic and/or filtration methods. A final solution containing the conjugate customarily may be lyophilized to provide the conjugate in a dry, stable form which can be safely stored and shipped. The lyophilized product eventually can be reconstituted with sterile water or another suitable diluent for administration. Alternatively, the ultimate product may be frozen, for example, with liquid nitrogen, and thawed and brought to ambient temperature prior to administration.

As disclosed by Willner et al, a first preferred embodiment the doxorubicin hydrazone of Formula (IIb) is made by reacting the doxorubicin with a maleimido-($C_1$-$C_{10}$)-alkyl hydrazide, or a salt thereof. The reaction generally is carried out in two steps. First the maleimido-($C_1$-$C_{10}$)-alkyl hydrazide, or its salt, is prepared. After purification by, for example, chromatography and/or crystallization, either the free base of the hydrazide or the salt is reacted with the doxorubicin salt. After concentration of the reaction solution, the maleimide-containing hydrazone reaction product of Formula (IIb) is collected, and if desired, purified by standard purification techniques.

The hydrazone then is reacted with a ligand, most preferably, an antibody, in which at least one disulfide bond has been reduced to form at least one sulfhydryl group. An especially preferred ligand is a "relaxed antibody", as described below. The preferred reducing agent for preparing a free sulfhydryl group is DTT.

A "relaxed" antibody, is one in which one or more, or preferably, four, disulfide bridges have been reduced. Most preferably, a relaxed antibody is one in which at least four disulfide bridges have been reduced. In a preferred process for preparing a relaxed (i.e. reduced) antibody, the reduction, especially with DTT, and the purification of the reaction product, is carried out in the absence of oxygen, under an inert atmosphere, for example, under nitrogen or argon.

As described by Willner et al, in an alternative procedure, the reaction is carried out at ambient conditions, however, a sufficiently large amount of the reducing agent, preferably DTT, is used to overcome any reoxidation of the reduced disulfide bonds which may occur. In either case, purification of the product, is carried out as soon as possible after the reaction is complete and most preferably under an inert atmosphere such as an argon or nitrogen blanket. The preferred method for preparing the free sulfhydryl containing ligand, however, is the process in which atmospheric oxygen is excluded from the reaction. An antibody produced by either method is referred to as a "relaxed" antibody. The product, however prepared, should be used for subsequent reaction as quickly as possible or stored under conditions which avoid exposure to oxygen, preferably under an inert atmosphere, and at about 0 °C.

In the process in which oxygen is excluded from the reaction (i.e. the reaction is performed under an inert atmosphere), the ligand (antibody) is incubated, for a period of about 30 minutes to about 4 hours, preferably about 3 hours, with a molar excess of DTT. The DTT/ligand ratios may range between about 1:1 to about 20:1, preferably about 4:1 to about 10:1, most preferably about 5:1 to about 7:1, depending upon

the number of sulfhydryl groups desired. For a reduction performed in the presence of oxygen, the mole ratio of DTT to ligand ranges from about 50:1 to about 400:1, preferably from about 200:1 to about 300:1. This latter reaction is carried out for about 1 to about 4 hours, preferably 1.5 hours, at a temperature of between about 20 °C and about 50 °C, with a preferred temperature being about 37 °C. The reaction is carried out at a pH of between about 6 and about 8, preferably between about 7 to 7.5. The product then is purified using standard purification techniques such as dialysis, filtration and/or chromatography. A preferred purification method is diafiltration. To prevent reoxidation of sulfhydryl groups, during purification and storage, the product preferably is maintained under an inert atmosphere to exclude exposure to oxygen at about 0 °C.

To prepare the final conjugate of Formula (Ia), the reduced antibody is reacted with the hydrazone intermediate of Formula (IIb). The reaction preferably is performed under an inert atmosphere at a temperature of about 0 °C to about 10 °C, preferably at about 4 °C and at a pH of about 6 to about 8, preferably about 7.5. The immunoconjugate or the thioether conjugate is purified using standard techniques such as dialysis, filtration, or chromatography.

In accordance with the present invention, a first method is provided for preparing a thioether conjugate in relatively pure form of the structure

I

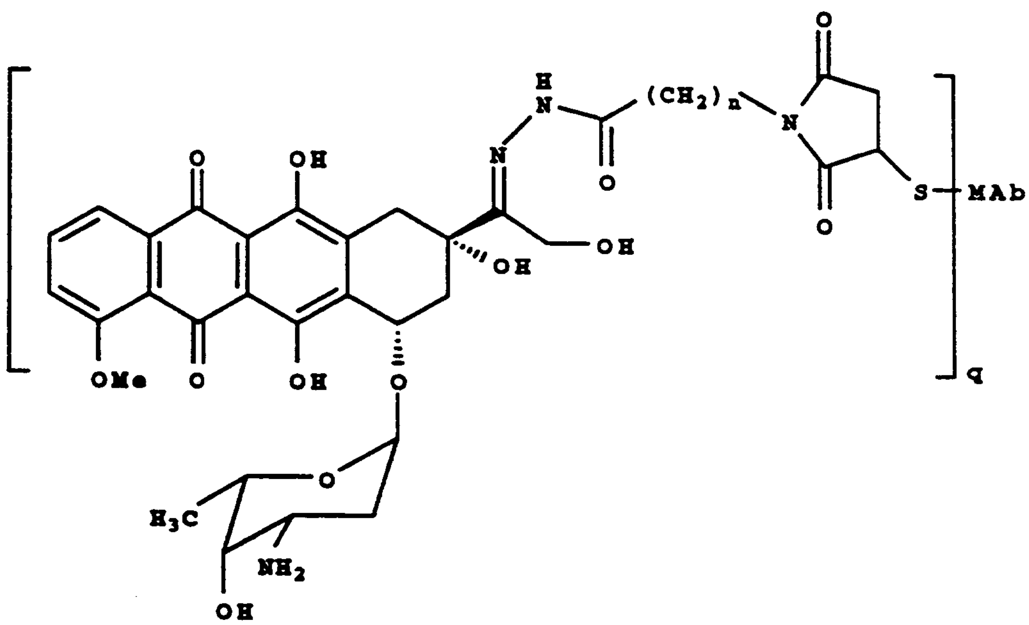

wherein n is an integer from about 1 to about 10;

q is an integer from about 4 to about 10, preferably 7-9; and

MAb is an immunoglobulin (monoclonal or polyclonal antibody, preferably monoclonal) ligand which includes a BR96 antibody;

which method includes the steps of

(a) providing an immunoglobulin thioether of the structure

II

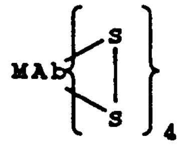

in a citrate buffer solution, the citrate buffer solution having a pH of within the range of from about 4 to about 6.5, preferably from about 5 to about 6;

(b) treating the citrate buffered immunoglobulin thioether (II) with a solution of phosphate buffer, the phosphate buffer solution having a pH of within the range of from about 6 to about 10, preferably from about 8 to about 10, to provide a buffered solution of immunoglobin thioether having a pH of within the range of from about 6 to about 9, preferably from about 7 to about 8;

(c) in a reduction step treating the buffered immunoglobulin thioether with a phosphate buffered solution of reducing agent, preferably dithiothreitol (DTT), having a pH of within the range of from about 6 to about 10, preferably from about 7 to about 8, under an inert atmosphere, such as argon, to form a buffered solution of reduced immunoglobulin thioether of the structure

III MAb-$(SH)_{6-9}$ (preferably 7-9)

having a pH of within the range of from about 6 to about 9, preferably from about 7 to about 8;

(d) diafiltering the solution of reduced form of immunoglobulin at reduced temperature, for example at from about -5 to about 25 °C, preferably at about 0 °C, under an inert atmosphere;

(e) optionally concentrating the buffered solution of reduced immunoglobulin during the diafiltration purification step;

(f) in a conjugation step reacting the diafiltered and purified solution of the reduced immunoglobulin with a solution of doxorubicin hydrazone of the structure

IV

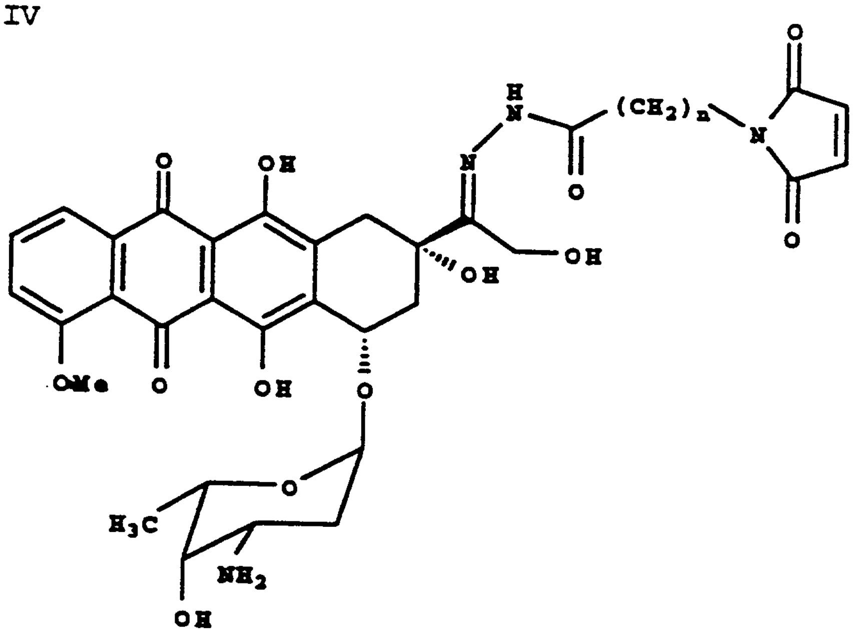

under an inert atmosphere to form crude thioether conjugate I, preferably carrying out the reaction under an argon atmosphere, for a period from about 10 to about 60 minutes, with the doxorubicin hydrazone being employed in an amount to provide from about 1.0 to about 1.5 equivalents for each thiol of the reduced immunoglobulin thioether;

(g) purifying the thioether conjugate I; and

(h) recovering the thioether conjugate I in relatively pure form.

The above procedure is outlined in reaction Schemes III and IV to be discussed in detail hereinafter.

In another embodiment of the present invention, a second method is provided for preparing a thioether conjugate I in relatively pure form, which method includes the steps of

(a) Diafiltering the citrate buffered immunoglobulin thioether II (of pH from about 5 to about 6) with a solution of phosphate buffer in a buffer exchange step, the phosphate buffer having a pH of within the range of from about 6 to about 10, preferably from about 6 to about 9, more preferably from about 7 to about 8, to provide a phosphate buffered solution of immunoglobin thioether having a pH of within the range of from about 6 to about 9, preferably from about 7 to about 8;

(b) optionally concentrating the solution of immunoglobulin thioether prior to or after the diafiltering step (a);

(c) in a reduction step treating the phosphate buffered solution of immunoglobulin thioether with a phosphate buffered solution of reducing agent, like dithiothreitol (DTT) or 2-mercaptoethanol, preferably dithiothreitol (DTT) (preferably a molar ratio MAb:DTT of 1:5), under an inert atmosphere such as argon, to form a buffered solution of reduced immunoglobulin III;

(d) reacting the phosphate buffered solution of reduced immunoglobulin III with a solution of doxorubicin hydrazone IV, under an inert atmosphere such as agron, to form crude thioether conjugate I;

(e) purifying the crude thioether conjugate I; and

(f) recovering the thioether conjugate I in relatively pure form.

The above reaction procedure is shown in reaction Schemes III and V to be discussed hereinafter.

In either of the methods outlined in Schemes IV and V, the phosphate-buffered solution of the immunoglobulin thioether may optionally be treated with a chelating agent, such as ethylenediaminetetraacetic acid, to remove metal impurities, if any, present in the buffer, immunoglobulin thioether and/or reaction vessel.

It is essential in the above methods that the pH of the immunoglobulin thioether II is adjusted to about 7 to about 8 prior to the reduction to III. The pH of the thioether II which is originally (with citrate buffer) within the range of from about 5 to about 6 is changed (with phosphate buffer) to within the range of from about 7 to about 8 by addition of phosphate buffer in the first method (scheme-IV) and the reduced thioether III obtained by this method undergoes a buffer exchange from citrate to phosphate

prior to or during reduction whereas in the second method (scheme-V) the pH of the thioether II which is originally (with citrate buffer) within the range of from abut 5 to about 6 is changed (with phosphate buffer) to within the range of from about 7 to about 8 in a diafiltration step prior to the reduction.

In addition, in accordance with the present invention, the crude thioether conjugate I is purified employing several different novel methods never before used to purify such a material.

In a first novel method of purification, crude thioether conjugate I is first subjected to filtration employing, for example, cellulose acetate membranes to clarify the solution, is then mixed with carbon or charcoal at a reduced temperature, and filtered as described in detail hereinafter.

In a second novel method of purification, crude thioether conjugate I is treated/purified with BIO-BEADS™ SM-2 (employed to enhance hydrophobic interaction, Bio-Rad Laboratories, Richmond, CA) in a batch process and then filtered out and recovered in substantially pure form.

In a third novel method of purification, crude thioether conjugate I is purified by column chromatography using gel filtration, ion-exchange or hydrophobic interaction. SEPHADEX™ G-25 (gel filtration medium) is the preferred medium for gel filtration; CM SEPHADEX™ C-25 is the preferred medium for ion-exchange, while BIO-BEADS™ are preferred for hydrophobic interaction.

In a fourth novel method of purification, crude thioether conjugate I is purified via diafiltration or dialysis preferably employing Amicon Stirred Cells with regenerated cellulose acetate membranes or Amicon CH2RS Dialyzer with regenerated cellulose acetate cartridges or Filtron Centrasette with polyether sulfone cassettes.

The purification may also be effected by passing the crude thioether conjugate I through polysulfone or hydrophilized polyacrylonitrile.

In addition, in accordance with the present invention a method is provided for preparing intermediates used in preparing the thioether conjugates of formula I, which method is as outlined in the following reaction Scheme I, and includes the steps of

(a) reacting maleic anhydride A with an amino acid of the structure

$H_2N-(CH_2)_n-COOH$ B

wherein n is an integer from 1 to about 10, preferably 5, in the presence of weak organic acid, preferably acetic acid, to form an acid condensation product C

C 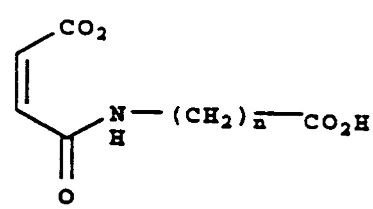 ;

(b) treating acid C with a cyclizing agent, such as a silylating agent, preferably chlorotrimethyl silane, in the presence of weak organic base, preferably triethylamine, and an inert organic solvent, preferably acetonitrile, to form D

D 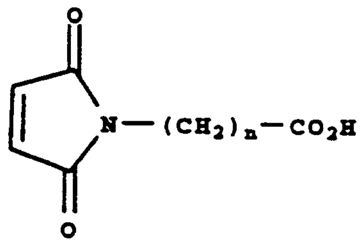

(which is preferably maleimidocaproic acid);

(c) subjecting D to crystallization to form cyclized crystallized acid D;

(d) subjecting the cyclized crystallized acid D to a coupling reaction by treating D with a coupling reagent, preferably isobutylchloroformate in the presence of N-methylmorpholine, and inert organic solvent such as tetrahydrofuran, and an alkylcarbazate, preferably, t-butylcarbazate, to form a carbazate E

E 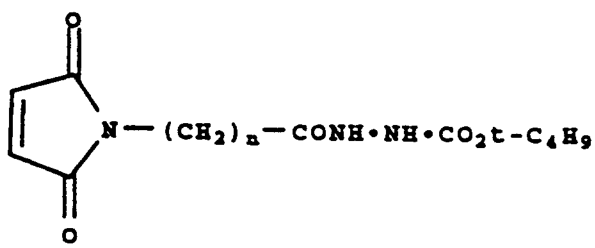 ;

and

(e) deprotecting carbazate E by treatment with acid, preferably trifluoroacetic acid, to form intermediate hydrazide salt F

F 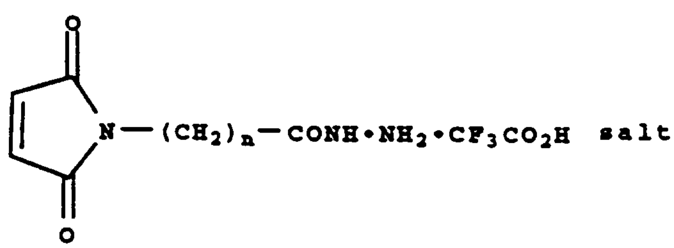

Alternatively, the acid D may be prepared by reacting a solution of acid B in glacial acetic acid or other acid such as formic acid or propionic acid, with a solution of maleic anhydride A in glacial acetic acid under an inert atmosphere such as argon or nitrogen, to directly form the acid D.

As will be seen in reaction Scheme II, the doxorubicin •HCl (G) is then condensed with hydrazide salt F as described in the Willner et al U.S. patent application Serial No. 824,951, filed January 23, 1992, to form the doxorubicin hydrazone IV.

The methods of the invention may be carried out as outlined in reaction Schemes I to V set out below wherein preferred embodiments of the invention are illustrated.

Scheme I

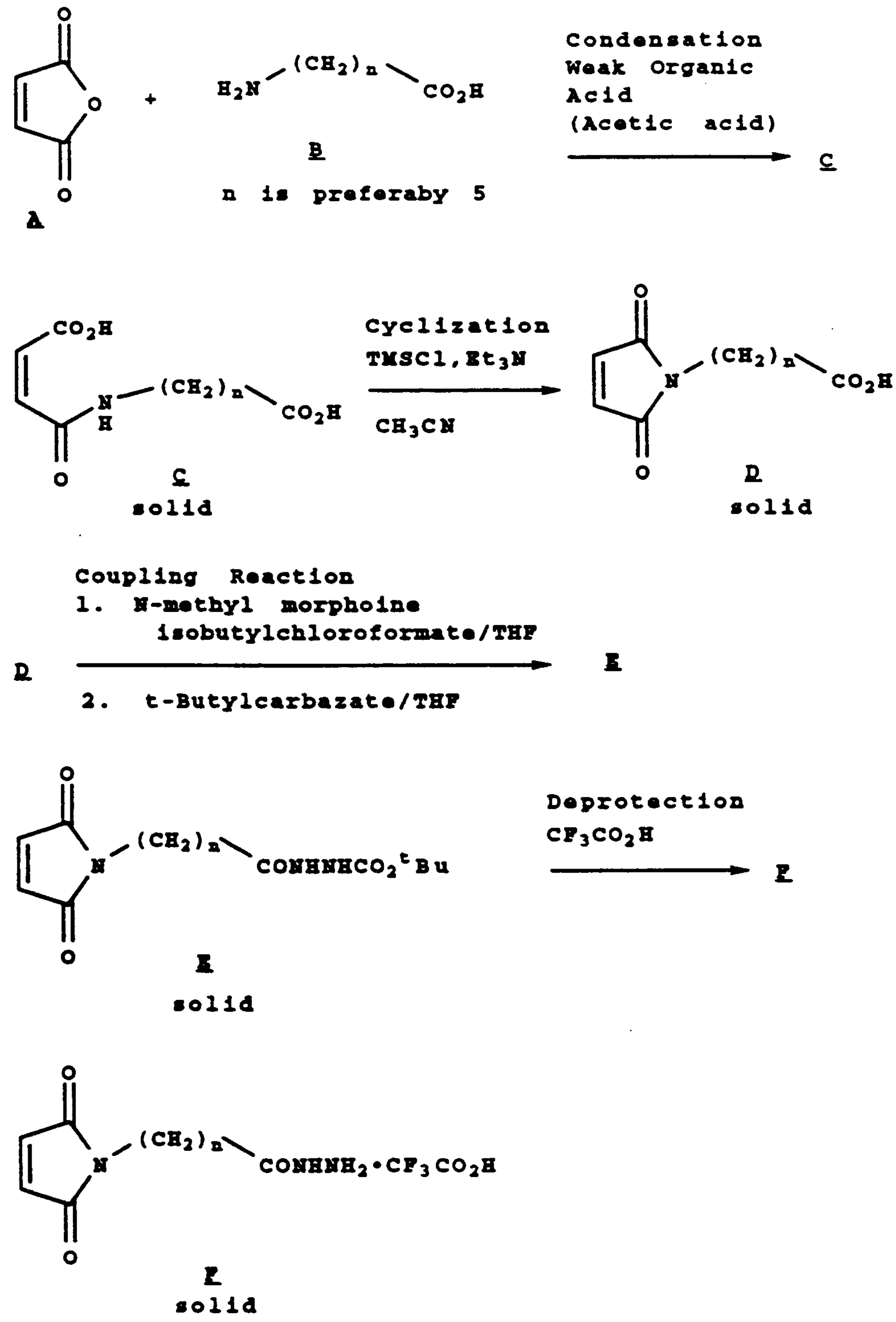
A
B
n is preferaby 5
Condensation
Weak Organic
Acid
(Acetic acid)
C
C
solid
Cyclization
TMSCl, Et3N
CH3CN
D
solid
D
Coupling Reaction
1. N-methyl morphoine
isobutylchloroformate/THF
2. t-Butylcarbazate/THF
E
Deprotection
CF3CO2H
F
E
solid
F
solid

Scheme II

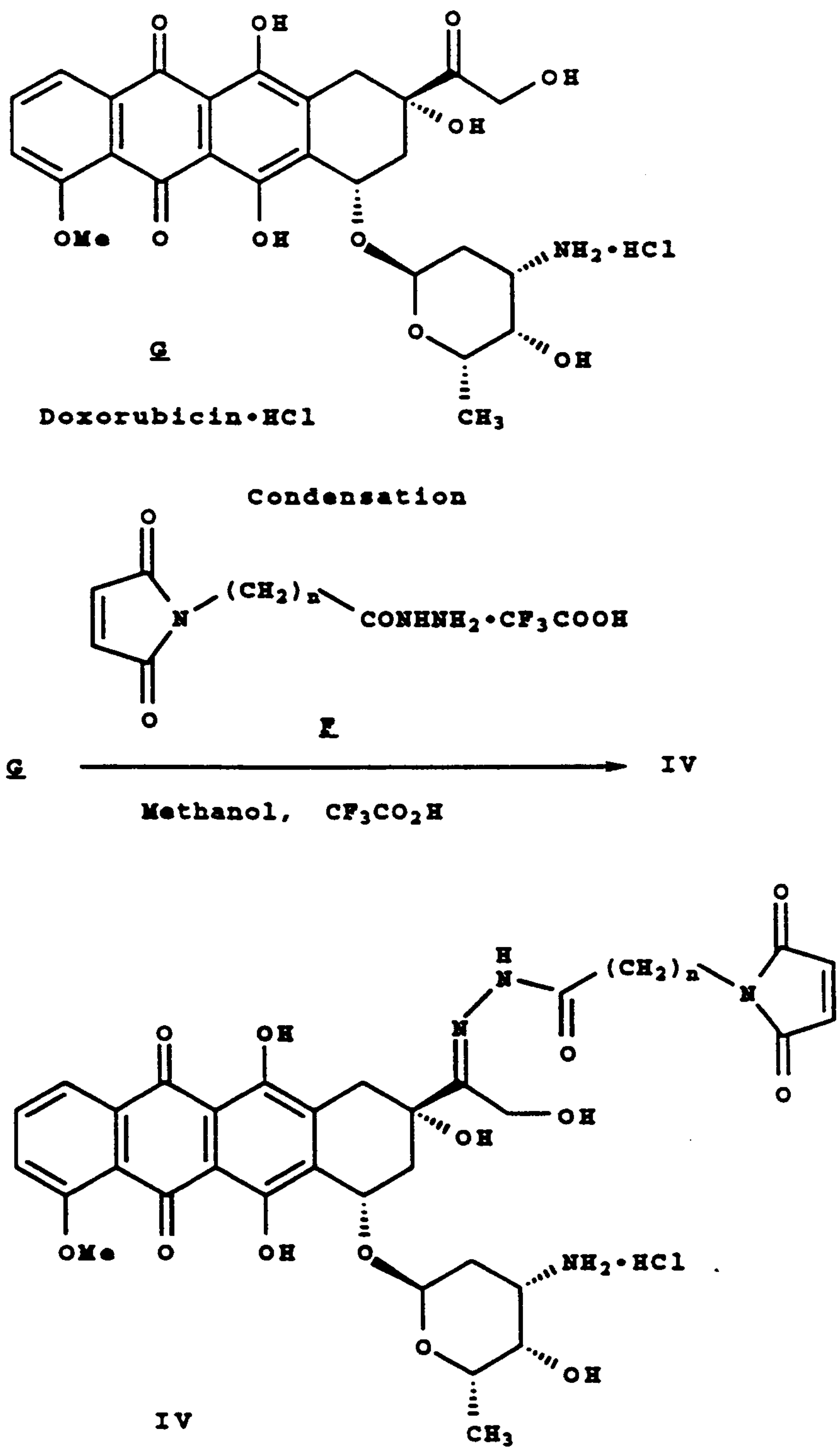

Scheme III

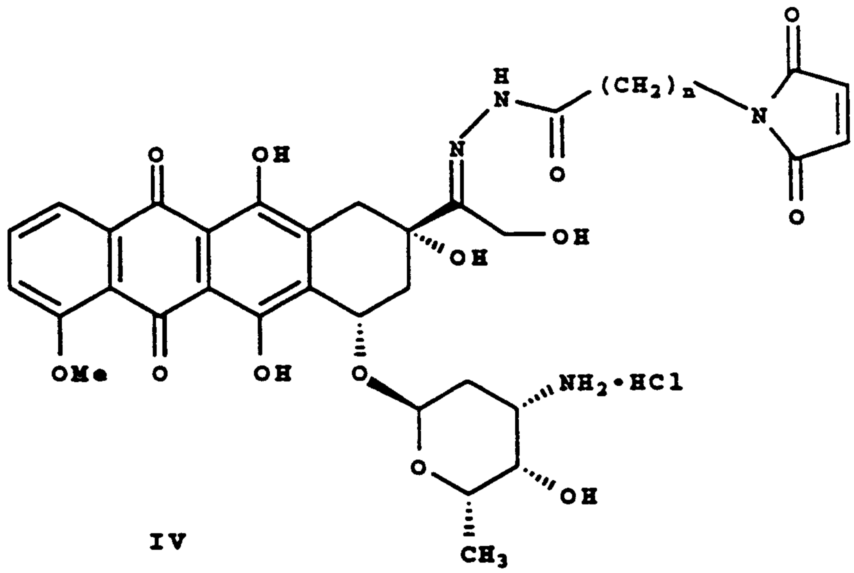

IV

Doxorubicin hydrazone

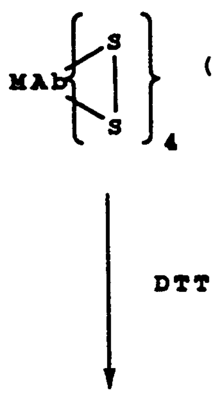


(II)

$MAb-(SH)_{7-9}$ (III)

Conjugate I

(Reduced antibody)

+ doxorubicin hyxazone (IV)

Conjugate I

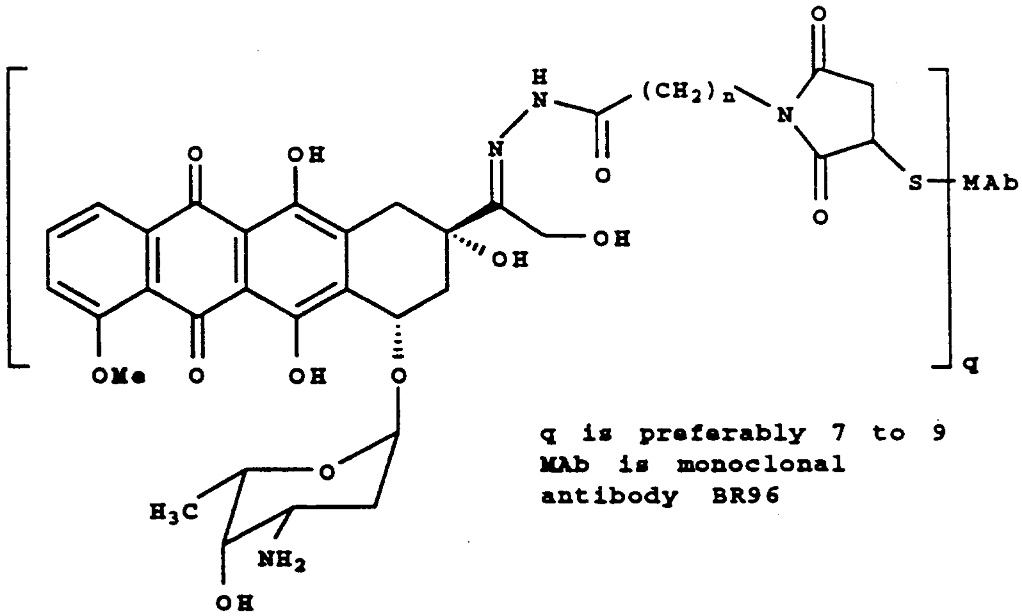

q is preferably 7 to 9
MAb is monoclonal antibody BR96

Scheme IV

CONJUGATION PROCEDURE (I)

BR96 MAb
pH 5.5 CBS Buffer

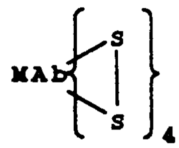
(II)

Reduction

1. pH to ~7.5 ($Na_2HPO_4$)

2. DTT (7.0 mole eq.), 37°C, 2 hours, argon atmosphere

Reduced
BR96 MAb
($MAb-(SH)_{7-9}$) (III)

Purification

Diafiltration (Filtron Cassettes)
Cold Room (4°C)
argon atmosphere

Purified & Reduced
BR96 MAb
($MAb-(SH)_{7-9}$) (III)

Conjugation

Doxorubicin hydrazone (IV)
(1.15 eq./each thiol group)
Cold Room (4°C)
20 minutes, argon atmosphere

Crude
Conjugate I

Purification

1. Polish filtration
(0.8 µm and 0.2 µm membranes)
2. Charcoal purification
OR
Chromatographic purification
OR
BIO-BEAD™ purification
OR
Diafiltration

Purified
Conjugate I

Scheme V

CONJUGATION PROCEDURE (II)

**BR96 MAb (II)**
**pH 5.5 CBS Buffer**

**Option for Concentration**

**Buffer Exchange**

**1. Diafiltration (Buffer exchange, from CBS to PBS**

**BR96 MAb (II)**
**pH 7.5 PBS Buffer**

**Reduction & Conjugation**

**1. DTT (5.0 mole eq.), 37°C, 4 hours**
**2. Doxorubicin hydrazone IV (1.15 eq./each thiol group of DTT) -4°C, 20 minutes, argon atmosphere**

**Crude Conjugate I**

**Purification**

**1. Polish filtration (0.8 µm and 0.2 µm membranes)**
**2. Charcoal, 15 min., 4°C**
**OR**
**Chromatographic purification**
**or**
**BIO-BEAD™ purification**
**or**
**Diafiltration**

**Purified Conjugate I**

As seen in reaction Scheme I, the starting hydrazide salt F is prepared by reacting a solution of maleic anhydride A in a weak acid, such as acetic acid, formic acid or propionic acid, preferably acetic acid, with a solution of amino acid B, which preferably is 6-aminocaproic acid (formula B where n is 5) in a weak organic acid, such as acetic acid or any of the other acids in which A is dissolved, preferably acetic acid, to afford the diacid C. The above reaction is carried out employing a molar ratio of B:A of within the range of from about 5:1 to about 0.5:1, preferably from about 2:1 to about 1:1, and a reaction time of within the range of from about 1 to about 10 hours, preferably from about 3 to about 5 hours.

Diacid C, dissolved in an inert organic solvent, such as acetonitrile, diethyl ether, tetrahydrofuran, dimethyl formamide or toluene, preferably acetonitrile, is treated with a cyclizing agent such as chlorotrimethyl silane (TMSCl), hexamethyldisilazane or similar silylating agent, preferably chlorotrimethyl silane, and a weak organic base, such as triethylamine, or diisopropylethyl amine, preferably triethylamine. The above cyclization reaction is carried out employing a molar ratio of cyclizing agent: diacid C of within the range of from about 10:1 to about 1:1, preferably from about 5:1 to about 3:1, at a temperature within

the range of from about 25 to about 110°C, preferably from about 50 to about 90°C, for a period of from about 1 to about 10 hours, preferably from about 3 to about 5 hours.

The resulting acid D, which preferably is maleimidocaproic acid, may be isolated in substantially pure form without the need for column chromatography. A preferred isolation procedure is crystallization, for example employing ethyl acetate and hexane, or acetone and isopropyl ether.

Alternatively, acid D may be prepared in one step by reacting a solution of maleic anhydride A in glacial acetic acid, formic acid or propionic acid, preferably glacial acetic acid, and a solution of amino acid B in glacial acetic acid, formic acid, propionic acid or acetic anhydride, preferably glacial acetic acid, under an inert atmosphere such as argon or nitrogen, preferably argon.

A and B may be employed in molar ratios as described above in the 2-step procedure for making D.

The reaction is carried out at a temperature within the range of from about 25 to about 150°C, preferably from about 100 to about 125°C, for a period of from about 1 to about 10 hours, preferably from about 3 to about 5 hours.

The cyclized acid D is made to undergo a coupling reaction to form carbazate E as follows: a solution of acid D in anhydrous inert organic solvent, such as tetrahydrofuran (THF), diethyl ether or acetonitrile, at a reduced temperature of within the range of from about -15 to about 15°C, preferably from about 0 to about 4°C, is treated with a solution of 4-methylmorpholine, triethylamine or diisopropyl ethylamine, in anhydrous solvent such as set out above for D, preferably THF, and a solution of alkylchloroformate such as isobutylchloroformate or pivaloyl chloride in anhydrous solvent such as set out above for D, preferably THF, at a temperature within the range of from about -5 to about 5°C, preferably from about 0 to about 4°C. Thereafter, a solution of alkylcarbazate, preferably t-butylcarbazate, in anhydrous solvent such as set out above for D, preferably THF, is added to the reaction mixture. The reaction is carried out at a temperature within the range of from about 0 to about 25°C, preferably from about 4 to about 25°C, for a period of from about 10 to about 30 hours, preferably from about 10 to about 24 hours. The 4-methylmorpholine or equivalent, alkylchloroformate and alkyl carbazate will each independently be employed in a molar ratio to acid D of within the range of from about 0.5:1 to about 5:1, preferably from about 2:1 to about 1:1. The carbazate E may be isolated by crystallization with ethyl acetate and hexane or other solvent mixture.

The resulting carbazate E is then deprotected by reacting E with an acid such as trifluoroacetic acid, hydrochloric acid, p-toluene sulfonic acid or hydrobromic acid, at a temperature within the range of from about -15 to about 25°C, preferably from about 0 to about 4°C, for a period of from about 1 to about 24 hours, preferably from about 2 to about 5 hours. The preferred maleimidocaproyl hydrazide TFA salt may be isolated by precipitation with t-butylmethyl ether or diethyl ether.

Referring to reaction Scheme II, the hydrazone of doxorubicin (IV) is prepared by reacting doxorubicin hydrochloride G, hydrazide salt F (preferably maleimidocaproyl hydrazide salt), and acid such as trifluoroacetic acid, hydrochloric acid or acetic acid, preferably trifluoroacetic acid in anhydrous alcohol such as methanol, ethanol or isopropanol preferably methanol, in the dark, under an inert atmosphere such as argon or nitrogen, preferably argon. The reaction is carried out for a period of from about 1 to about 10 hours, preferably from about 3 to about 5 hours.

The doxorubicin hydrazone product IV may be isolated by direct precipitation by addition of ethyl acetate, acetonitrile or tetrahydrofuran, at a reduced temperature, for example, within the range of from about 0 to about 10°C, preferably from about 2 to about 4°C, and collected by filtration and the product washed with cold solvent such as methanolethyl acetate mixture followed by acetonitrile and dried under vacuo.

Referring now to reaction Schemes III and IV, the doxorubicin BR96 conjugate of structure I is prepared in a first embodiment of the method of the invention as follows: A solution of alkali metal phosphate sodium phosphate dibasic (prepared by dissolving $Na_2HPO_4$ in water for injection (WFI)) having a pH of within the range of from about 8 to about 10, preferably from about 8.5 to about 9.5, is added to a solution of monoclonal antibody BR96 (formula II) in citrate buffer (CBS) (preferably from about 10 to about 75 mM sodium citrate and from about 50 to about 500 mM sodium chloride). The citrate buffer (CBS) will have a pH of within the range of from about 4 to about 6.5, preferably from about 5 to about 6.5, more preferably from about 5 to about 6, to provide a solution of BR96 MAb having a pH of within the range of from about 6 to about 9, preferably from about 7 to about 8. The resulting solution of BR96 MAb is purged with argon to reduce $O_2$ content to less than about 10%, preferably less than about 5%.

A solution of reducing agent such as dithiothreitol (DTT), or 2-mercaptoethanol, preferably dithiothreitol (DTT), in phosphate buffer solution (PBS) is prepared. The PBS buffer will have a pH of within the range of from about 6 to about 10, preferably from about 7 to about 8, and will be formed of from about 1 to about 25 mM sodium phosphate and from about 50 to about 250 mM of sodium chloride (from about 0.005M to about 0.02M sodium phosphate and from about 0.05M to about 0.2M sodium chloride prepared

from about 1 to about 5 mM of $NaH_2PO_4 \cdot H_2O$,
from about 5 to about 15 mM of $Na_2HPO_4$,
from about 50 to about 200 mM of NaCl
water Qs to one liter
1N NaOH added till pH of from about 7.0 to about 8.0 is attained).
Alternatively, the phosphate buffer may be formed of
from about 50 to about 200 mM (from about 0.04 to about 0.4% by weight) KCl,
from about 5 to about 15 mM (from about 0.4 to about 4% by weight) $Na_2HPO_4$
from about 1 to about 5 mM (from about 0.04 to about 0.4% by weight) $KH_2PO_4 \cdot H_2O$
water Qs to one liter,
1N KOH added to attain pH of from about 7 to about 8.

The solution of reducing agent, preferably DTT in phosphate buffer, is reacted with the solution of BR96 MAb II, in a molar ratio reducing agent:II of within the range of from about 10:1 to about 5:1, preferably from about 9:1 to about 6:1, at a temperature of from about 25 to about 45°C, preferably from about 30 to about 40°C, for a period of from about 1 to about 5 hours, preferably from about 2 to about 3 hours. The above reaction is carried out under an inert atmosphere, preferably argon.

The resulting reduced BR96 MAb

III MAb-$(SH)_{7-9}$

is mixed with argon-purged phosphate buffer and then is preferably subjected to diafiltration/concentration under argon atmosphere using Waters, Amicon or Filtron diafiltration system to remove impurities.

The purified and reduced BR96 MAb is next subjected to conjugation with doxorubicin hydrazone IV under an inert atmosphere such as argon, at a temperature of from about -5 to about 25°C, preferably from about 0 to about 5°C, employing a molar ratio of reduced BR96 MAb:IV of within the range of from about 1:4 to about 1:11, preferably from about 1:8 to about 1:10.

Referring to reaction Schemes III and V, the doxorubicin BR96 conjugate of structure I is prepared in a second embodiment of the method of the invention as follows:

A solution of BR96 MAb (II) in citrate buffered saline (CBS) (described with respect to the first embodiment of the method of the invention), the citrate buffer having a pH of within the range of from about 4 to about 6 , preferably from about 5.0 to about 5.5, is subjected to diafiltration with phosphate buffer (PBS) (described with respect to the first embodiment of the method of the invention) using a dialysis membrane, as described herein, preferably Filtron Minisette employing a 1:5 ratio of citrate buffered BR96 MAb: phosphate buffer of within the range of from about .5:1 to about .125:1, preferably from about .33:1 to about .2:1. The resulting phosphate buffered BR96 MAb (II) with a concentration of about 5 to 15 mg/ml, is treated with reducing agent, preferably a solution dithiothreitol (DTT) in phosphate buffer employing a molar ratio of II:DTT of within the range of from about 0.1:1 to about 1:1, preferably from about 0.12:1 to about 0.2:1. The above reaction is carried out at a temperature of from about 25 to about 45°C, preferably from about 30 to about 40°C, under an inert atmosphere such as argon, for a period of from about 1 to about 6, preferably from about 3 to about 4 hours.

The reduced BR96 MAb (III) is then reacted with doxorubicin hydrazone IV as described herewith with respect to the first embodiment (Scheme IV) to form crude conjugate I which may be purified as described herein.

As will be apparent from the above, in the first embodiment of the method of the invention, after the immunoglobulin thioether II is reduced, the reduced antibody III is subjected to buffer exchange (treatment with phosphate buffer) and purification before conjugation with the doxorubicin hydrazone IV, while in the second embodiment of the method of the invention, after the immunoglobulin thioether II is reduced to III, the conjugation step with hydrazone IV proceeds directly.

The second embodiment of the method of the invention may include an optional concentration of the citrate buffered immunoglobulin thioether II which is carried out by continuing the diafiltration (dialysis) step for as long as necessary to achieve the desired concentration preferably within the range of from about 10 mg/ml to about 30 mg/ml immunoglobulin thioether.

It should also be noted that in the second embodiment, less reducing agent (DTT) may be employed, but time for complete reaction will be increased.

In accordance with the present invention, the resulting crude conjugate I from Schemes IV and V may be purified by any of the techniques described herein including using charcoal, column chromatography, dialysis and/or BIO-BEADS™. For example, the crude conjugate my be clarified by filtering through cellulose acetate membranes and then treated with charcoal under an inert atmosphere such as argon, at a

temperature within the range of from about 0 to about 20 ° C, preferably from about 0 to about 5 ° C.

Examples of charcoals suitable for use herein include the following:

1. NORIT™ SX-3 (preferred)
2. DARCO™ G60
3. ADP™ PULVERIZED
4. NORIT™ A SUPRA
5. NORIT™ B SUPRA
6. NORIT™ USP 22

Alternatively, in accordance with the present invention, the crude conjugate I may be purified by diafiltration or dialysis employing any of the following:

<u>Diafiltration</u>

1. Small Scale (<3.0 gram scale)

   a. Amicon Stirred Cells with Regenerated Cellulose Acetate Membranes
   b. Filtron Stirred cells with Polyether Sulfone Membranes

2. Large Scale (>3.0 grams scale)

   a. Amicon CH2RS Dialyzer with regenerated cellulose acetate Spiral cartridge - preferred
   b. Amicon DC10L Dialyzer with regenerated cellulose acetate Spiral cartridge
   c. Filtron Minisette with polyether sulfone Cassettes
   d. Filtron Centrasette with polyether sulfone Cassettes

The diafiltration of the conjugate I will be conducted under an argon atmosphere at a temperature of within the range of from about 0 to about 25 ° C,.

In addition, in accordance with the present invention, the crude conjugate I may also be purified by use of BIO-BEAD™ employed in column chromatography or preferably a batch process wherein the crude conjugate and preferably BIO-BEAD™ SM-2 (made of polystyrene divinylbenzene-Bio-Rad Laboratories) are stirred together under an inert atmosphere such as argon at a temperature of from about 0 to about 25 ° C, preferably from about 0 to about 5 ° C, for a period of from about 5 to about 120 minutes, preferably from about 10 to about 30 minutes.

Further, in accordance with the present invention, the crude conjugate I may be purified employing column chromatograhy using

I. Gel Filtration with any of the following gels

   1. SEPHADEX™ G-25 MEDIUM
   2. SEPHADEX™ G-50 MEDIUM
   3. SEPHADEX™ G-25 SUPER FINE (SF)
   4. SEPHACRYL™ S-100 HR
   5. SUPEROSE™ 12 PG
   6. ULTRAGEL™ AcA 202
   7. TRYSACRYL™ GF 05;

II. Ion-Exchange with any of the following gels

   1. CM SEPHAROSE™ FAST FLOW
   2. CM SEPHADEX™ G-25
   3. CM SEPHADEX™ G-50; or by

III. Hydrophobic Interaction with any of the following

   1. BIO-BEADS™ SM-2
   2. BIO-BEADS™ SM-7
   3. BIO-BEADS™ SM-16
   4. PHENYL SEPHAROSE™ 6 FAST FLOW

CM SEPHADEX™ C-50 SEPHADEX™ G-25 SF and CM SEPHADEX™ C-25 are preferred for the purification of the conjugate I.

CM SEPHADEX™ C-25 is particularly preferred for large scale purification, for example, on a 5.0 gram or larger scale.

Purification of conjugate I by column chromatography is carried out as follows: The crude conjugate I in phosphate buffer (pH of within the range of from about 7 to about 8) is loaded in a column at a temperature of from about 0 to about 5°C. The conjugate is eluted with phosphate buffer at a volumetric flow rate of from about 45 to about 85 ml/min.

With regard to preferred embodiments, the most preferred aspect of the method of the invention is the preparation of an immunoconjugate of Formula (I) in which the drug moiety is adriamycin (doxorubicin) and the ligand portion is selected from BR96 MAb, relaxed BR96 antibody chimeric BR96 MAb, and the antigen-recognizing fragments thereof. The most preferred ligand for this embodiment is chimeric BR96 MAb, especially relaxed chimeric BR96 MAb, and the antigen-recognizing fragments thereof.

The conjugates prepared by the method of the invention are capable of targeting a selected cell population and may be employed to treat the same disease states as doxorubicin (adriamycin) or derivatives thereof, including neoplastic diseases such as cancer, viral or other pathogenic infections, autoimmune disorders and other disease states in which adriamycin is used.

The conjugates prepared by the method of the invention are administered to the patient in the form of a pharmaceutical formulation which comprises a conjugate of Formula (I) and a pharmaceutically acceptable carrier, excipient or diluent therefor. As used, "pharmaceutically acceptable" refers to those agents which are useful in the treatment or diagnosis of a warm-blooded animal including, for example, a human, equine, porcine, bovine, murine, canine, feline, or other mammal, as well as an avian or other warm-blooded animal. The preferred mode of administration is parenterally, particularly by the intravenous, intramuscular, subcutaneous, intraperitoneal, or intralymphatic route. Such formulations can be prepared using carriers, diluents or excipients familiar to one skilled in the art. In this regard, See, e.g. Remington's Pharmaceutical Sciences, 16th ed., 1980, Mack Publishing Company, edited by Osol et al. Such compositions may include proteins, such as serum proteins, for example, human serum albumin, buffers or buffering substances such as phosphates, other salts, or electrolytes, and the like. Suitable diluents may include, for example, sterile water, isotonic saline, dilute aqueous dextrose, a polyhydric alcohol or mixtures of such alcohols, for example, glycerin, propylene glycol, polyethylene glycol and the like. The formulations may contain preservatives such as phenethyl alcohol, methyl and propyl parabens, thimerosal, and the like. If desired, the formulation can include 0.05 to about 0.20 percent by weight of an antioxidant such as sodium metabisulfite or sodium bisulfite.

For intravenous administration, the formulation preferably will be prepared so that the amount administered to the patient will be from about 0.01 to about 1 g of the desired conjugate I. Preferably, the amount administered will be in the range of about 0.2 g to about 1 g of the conjugate I. The conjugates I are effective over a wide dosage range depending on factors such as the disease state to be treated or the biological effect to be modified, the manner in which the conjugate is administered, the age, weight and condition of the patient as well as other factors to be determined by the treating physician. Thus, the amount administered to any given patient must be determined on an individual basis.

One skilled in the art will appreciate that although specific reagents and reaction conditions are outlined in the following Examples, modifications can be made which are meant to be encompassed by the spirit and scope of the invention. The following Examples however illustrate preferred embodiments of the invention.

## Example 1

### 2,5-Dihydro-2,5-dioxo-1H-pyrrole-1-hexanoic acid

### A. (Z)-6-[(3-Carboxy-1-oxo-2-propenyl)amino]hexanoic acid

A solution of maleic anhydride (15.0 grams, 0.15 mol) in glacial acetic acid (100 ml) was added at RT to a solution of 6-aminocaproic acid (19.65 grams, 0.15 mole) in glacial acetic acid (100 ml) in about 15 minutes and stirred for 3 hours at RT. The white precipitate was filtered, washed with cold water and dried in vacuum oven at 50° C for 2 days to afford 33.00 grams of title compound in 95% yield. This material was used in the next step without any further purification. m.p.: 170-172° C. TLC: $R_f$ = 0.47, silica gel, MeOH-$CHCl_3$ 1:1, visualized by PMA spray.

B. 2,5-Dihydro-2,5-dioxo-1H-pyrrole-1hexanoic acid

To a solution of Part A compound (3 grams, 0.013 mole) in acetonitrile (90 ml) were added dropwise at RT chlorotrimethyl silane (8.3 ml, 0.065 mole) and triethylamine (9 ml, 0.06 m mol) sequentially. The homogenous mixture was then heated at reflux temperature for 7 hours. After cooling to RT, the precipitated solid (triethylamine hydrochloride salt) was filtered and the filtrate was concentrated under vacuum to give a brown solid. The residue was dissolved in dichloromethane (75 ml) and boiled with neutral Norit (1.5 grams) for 20 minutes. It was filtered through a bed of celite and washed with hot dichloromethane (25 ml). The combined filtrate was concentrated in vacuum to afford 1.4 grams (50%) of the product. The crude product was crystallized using acetone and isopropyl ether (1:2) to give 0.91 grams of title compound in 32% yield. m.p.: 83-85 ° C. TLC: $R_f$ = 0.26, silica gel, MeOH-$CHCl_3$ 5:95, visualized by PMA spray.

Example 2

6-Maleimidocaproylhydrazone of Doxorubicin (Dox)

A. 2,5-Dihydro-2,5-dioxo-1H-pyrrole-1-hexanoic acid

A solution of 6-aminocaproic acid (20 grams, 0.153 mol) in glacial acetic acid (100 ml) was added at RT to a solution of maleic anhydride (15.0 grams, 0.153 mol) in glacial acetic acid (300 ml) under argon over a period of 10 minutes. After stirring at RT for 3 hours, the white heterogeneous reaction mixture was refluxed for 5 hours. Most of the acetic acid was removed on a rotary evaporator at 50 ° C under vacuum. Residual acetic acid was removed by codistillation with toluene under reduced pressure. The brown residue (40 grams) was dissolved in dichloromethane (250 ml) and boiled with neutral charcoal (20 grams) for 20 minutes. The hot mixture was filtered through a bed of celite and washed with hot dichloromethane (2 x 75 ml). The combined filtrate was concentrated in vacuum to yield 33 grams of crude product as a tan solid. The crude product was crystallized from EtOAc and hexane (1:1) to afford 13 grams of the product in three crops. It was recrystallized from EtOAc and hexane (1:1) to yield 9.8 grams of maleimidocaproic acid in 30% yield: m.p.: 82-84 ° C; TLC: $R_f$ = 0.25, silica gel, MeOH-$CHCl_3$ (5:95), visualized by PMA spray.

B. 2-[6-(2,5-Dioxo-1H-pyrrol-1-yl)-1-oxohexyl]hydrazinecarboxylic acid, 1,1-dimethylethyl ester

To a solution of Part A compound (15.0 grams, 70 m mol) in anhydrous THF (500 ml) at 0-4 ° C was added sequentially a solution of 4-methylmorpholine (7.7 ml, 70 m mol) in anhydrous THF (20 ml) and a solution of isobutylchloroformate (9.1 ml, 70 m mol) in anhydrous THF (20 ml). After stirring at 0 ° C for 15 min., a solution of t-butyl carbazate (9.24 g, 70 m mol) in anhydrous THF (30 ml) was slowly added (about 10 minutes) to the reaction. The yellowish heterogeneous mixture was stirred at 0 ° C for 1 hour and at RT for 24 hours. The progress of the reaction was monitored by TLC. The reaction mixture was concentrated to dryness. The residue was dissolved in EtOAc (200 ml) and washed sequentially with water (2 x 100 ml), ice cold 0.01 N HCl (100 ml), 5% sodium bicarbonate (100 ml) and water (2 x 100 ml). It was dried (anhydrous $MgSO_4$), filtered and concentrated on a rotary evaporator to give the crude product as a yellow oil (19.75 grams) which was purified by flash chromatography (silica gel 230-400 mesh) eluting sequentially with 35% EtOAc/hexane, 50% EtOAc/hexane and 75% EtOAc/hexane. The appropriate fractions were pooled and concentrated under vacuum to afford 19.6 grams (85%) of the title carbazate as a viscous oil: TLC: $R_f$ = 0.28 (S.M. $R_f$ = 0.35) silica gel, EtOAc-hexane (7:3), visualized by PMA spray.

C. 2,5-Dioxo-1H-pyrrole-1-hexanoic acid, hydrazide, trifluoroacetate salt

The Part B carbazate (16.9 grams) was dissolved in trifluoroacetic acid (60 ml) at 0 ° C and stirred for 3 hours at the same temperature. The excess trifluoroacetic acid was removed on a rotary evaporator at 30 ° C under high vacuum. The oily residue was triturated with ether (150 ml) at 0 ° C and allowed to stir for 2 hours at the same temperature. The white precipitate was filtered, washed with ether (50 ml) and dried under high vacuum overnight to give 15.0 grams (65.3% from Part A compound) of Part C compound: mp: 107-110 ° C, TLC: $R_f$ = 0.35 (S.M. $R_f$ = 0.60), silica gel, MeOH-$CH_2Cl_2$ (1:9), visualized by UV and PMA spray.

D. 6-Maleimidocaproylhydrazone of Doxorubicin

A mixture of doxorubicin hydrochloride (5.0 grams, 8.6 m mol), Part C maleimidocaproyl hydrazide salt (8.75 grams, 25.81 m mol), and trifluoroacetic acid (0.5 ml, 6.49 m mol) in anhydrous methanol (1.8 L) was stirred in the dark at RT under argon for 16 hours. The progress of the reaction was monitored by reverse phase HPLC. After near completion of the reaction, the mixture was directly concentrated, in dark, on a rotary evaporator at 30 °C under reduced pressure to about 200 ml. Anhydrous acetonitrile (2 L) was slowly added while stirring to the methanolic solution of the product to precipitate title compound. It was kept at 0-4 °C for 12 hours for complete precipitation. The product was filtered on a Buchner funnel with medium pore fritted disc. The dark red product was washed with a mixture of cold $MeOH-CH_3CN$ (1:9; 500 ml) and dried in vacuum for 3 to 4 hours to afford 6.36 grams (93%) of title doxorubicin hydrazone. The above product was further purified by dissolving in methanol (about 200 ml) and reprecipitating with anhydrous acetonitrile (2 L) as before. Isolation after filtration and drying afforded 5.42 grams (82%) of title compound: mp: 182-184° C, TLC: $R_f$ = 0.46, silica gel, MeOH-CHCl3-AcOH (3:7:0.1), 2 elutions, visualized by UV, PMA and AA spray, HPLC: HI 95.4% ($R_t$ = 15.92), II 2.9% doxorubicin hydrochloride.

Example 3

6-Maleimidocaproylhydrazone of Doxorubicin

To a cooled (ice-water bath) and stirred red suspension of doxorubicin hydrochloride, (10.0 grams, 17.24 mmol) in anhydrous methanol (400 ml) in a 5 L three-necked round bottomed flask, was added sequentially the Example 2, Part C maleimidocaproylhydrazide trifluoroacetic acid salt (17.53 grams, 51.72 mmol) in one portion followed by trifluoroacetic acid (8 $\mu$l, 0.1 mmol ) in anhydrous methanol (40 $\mu$l). The resulting mixture was stirred at the same temperature for 5 hours. After the completion of the reaction, cold (0-4 °C) ethyl acetate (4 L) was carefully added while stirring gently through an addition funnel over a period of 3 hours keeping the temperature between 0-4 °C. The red precipitate was filtered on a Buchner funnel with medium pore fritted disc. The bright red solid was washed with a mixture of cold (0-4 °C) MeOH-EtOAc (1:9; 6 x 200 ml) followed by acetonitrile (6 x 200 ml). The product was transferred into a 500 ml beaker and dried *in vacuo* (~0.3 mmHg) for 24 hours to afford 13.23 grams of title doxorubicin hydrazone. The above product was further dried *in vacuo* (~0.3 mmHg, 45 °C) for 24 hours to afford 13.13 grams in 97% yield with HI 99.4%, IR 0.4%, (doxorubicin), and 0.2% (unknown impurity).

Example 4

Doxorubicin-BR96 Conjugate (BR96 Monoclonal Antibody Conjugated to 6-Maleimidocaproylhydrazone of Doxorubicin)

1. Reduction of BR96 with DTT to form BR96 $MAb(SH)_{7-9}$

A saturated solution of sodium phosphate (dibasic, ~2155 ml, pH ~9.0) was added in about 20 minutes to a mechanically stirred solution of monoclonal antibody BR96, 6000 ml, conc. = ~10 mg/ml, in citrate buffered saline (CBS buffer, 25 mM sodium citrate and 250 mM sodium chloride, pH = 5.5), in a three necked 12 L round bottom flask to adjust the final pH to 7.71. This solution was constantly purged with ultra pure sterile filtered argon for about one hour. At the end of purging, the dissolved oxygen content in the antibody solution was less than 5%. The antibody concentration (7.327 mg/ml, 59.76 grams, 0.3734 mmol) was determined[a]. To this, a solution of dithiothreitol (DTT) (262.5 ml, 0.01 M in PBS[b], 2.625 mmol, 7.03 mole equiv.) was added while stirring in about five minutes at RT. The reaction mixture was then stirred gently in a water bath at 36-38 °C (internal temp.) for 2 hours under argon atmosphere to complete the reduction. The reduced antibody solution was transferred to a cold room (4 °C) for diafiltration.

Notes:

a. The antibody concentration (mg/ml) was determined using Perkin Elmer Lambda 6 UV/visible spectrophotometer and the UV absorption at 280 nm; 1 mg/ml = 1.4 absorbance units.

Molar concentration of antibody was calculated as follows:

$$MC_{antibody} = \frac{mg/ml}{160000}$$

where 160000 is the molecular weight of the antibody.

b. PBS buffer , pH = 7.8 (0.01 M sodium phosphate and 0.1 M sodium chloride) was prepared as follows.

| | |
|---|---|
| $NaH_2PO_4 \cdot H_2O$ | 0.14 grams |
| $Na_2HPO_4$ | 1.28 grams |
| NaCl | 5.85 grams |
| Water (WFI) | QS to one liter |
| 1 N NaOH | ~0.5 ml (added till pH was 7.8) |
| Filtered through a 0.22μ cellulose acetate membrane. | |

2. Diafiltration of Reduced BR96

The Part 1 reduced antibody was transferred into a 20 L polypropylene carboy with a spigot containing three liters of argon purged PBS buffer by pumping with a perilstatic pump under argon atmosphere. The resulting solution (~11 L) in the carboy was concentrated under argon atmosphere using Filtron Centrasette[c] diafiltration/concentration unit to about 6 L. The concentrated solution (6 L) was then diafiltered while gently stirring with a magnetic stirrer with 24 L of argon purged PBS buffer. The flow rate of the filtrate was about 1.1 L/min. After a total of five volumes (30 L) of diafiltration, the reduced antibody (5520 ml) was transferred to a three necked 12 L round bottom Morton flask under argon atmosphere. The Molar Concentration of the SH[d] and the antibody[a] was determined.

Notes:

c. The Centrasette diafiltration unit made of 316L stainless steel was purchased from Filtron. Two Omega 30 K membranes (made of polyethersulfone) with 10 sq. feet total surface area were used. The antibody solution was pumped into the unit from the reservoir with Watson-Marlow 604 S/R perilstatic pump attached with 15.9 mm Bioprene tubes. The pressure was less than 20 psi.

d. The molarity of the thiol group was determined according to the Ellman method (Anal. Biochem. 94, 75-81, 1979). The reaction mixture (0.1 ml) was diluted to 1 ml with PBS buffer and treated with 0.025 ml of Ellman reagent ( a 50 m molar solution of 5,5'-dithio-bis-2-nitrobenzoic acid, DTNB, in 100 m molar sodium phosphate dibasic, pH 7.00). A blank of 1 ml PBS buffer was treated with 0.025 ml of DTNB in the same manner. After 5 minutes the absorbance (A) of the sample and the blank were measured at 412 nm and the molar concentration of thiol ($MC_{SH}$) was determined according to the equation below.

$$MC_{SH} = \frac{(A_{sample} - A_{blank}) \times 10}{1.415 \times 10^4}$$

where 10 is the dilution factor and $1.415 \times 10^4$ is the molar absorption coefficient of the dianion of 2-nitro-5-thiobenzoic acid.

The Molar ratio (MR) of thiol group to antibody was calculated as follows

$$MR = \frac{MC_{SH}}{MC_{antibody}}$$

3. Conjugation of Reduced BR96

To the mechanically stirred solution of the above reduced and diafiltered antibody, an aqueous (WFI) solution of doxorubicin hydrazone (such as prepared in Example 3) (611 ml $H_2O$, 3.68 mmol, 3.05 g, HI 95%) was slowly added in about 10 minutes under argon at 5 ° C. After stirring for 20 minutes at the same temperature, the crude product was clarified by pumping through two capsules of 0.8μ and 0.2μ cellulose acetate membranes. To the stirring solution of the crude product in a three necked 12 L round bottom flask, 30 grams of charcoal was added in one portion and stirred for 15 minutes at 5 ° C under argon[1]. The product was filtered through two vacuum filtration units (1L Corning Vacuum Filtration unit, 0.45μ followed by 0.22μ). The title product, (6350 ml) was temporarily stored in a 10 L polypropylene carboy with a spigot. The antibody concentration and molarity of the doxorubicin ( and hence the MR of antibody to doxorubicin) was then determined.[2] The pH of the conjugate was 7.48, antibody concentration was 9.1 mg/ml and the molar ratio was 8.49.

The above conjugate was divided into two portions:

Portion I: 2830 ml

Portion II. 3520 ml (This portion was used in Example 11).

Portion I was then frozen as 28 x 100 ml and 5 x 5 ml portions in sterile plastic bottles in liquid nitrogen and stored at -80-85 ° C.

Notes:

1. The charcoal (Fisher Scientific, Cat # C170-500, Norit, neutral) was washed while stirring with PBS buffer (pH 7.4) until the pH of the washings were constant at 7.4 and dried in vacuum oven.

2. The concentration of the antibody (mg/ml) in the conjugate was determined as before by absorbance at 280 nm with a correction for the absorbance of doxorubicin at the same wavelength according to the equation below.

$$\frac{A_{280} - (0.724 \times A_{495})}{1.4}$$

where 0.724 is the correction factor for doxorubicin absorbance at 280 nm and 1.4 is the absorbance unit for the antibody.

The molar concentration of the doxorubicin was determined by the equation given below

$$MC_{doxorubicin} \quad \frac{A_{495}}{8.03 \times 10^3}$$

where $8.03 \times 10^3$ is the molar absorption coefficient of doxorubicin at 495 nm.

The Molar Ratio (MR) of the conjugate was calculated by the following equation

$$\frac{MC_{doxorubicin}}{MC_{antibody}}$$

Example 5

Doxorubicin-BR96 Conjugale (BR96 Monoclonal Antibody Conjugated to 6-Maleimidocaproylhydrazone of Doxorubicin)

To a solution of monoclonal antibody BR96, (325 ml, conc. = 9.73 mg/ml in citrate buffered saline[a.]) in a sterile plastic bottle was added dropwise a solution of dithiothreitol (DTT) (13.84 ml, 0.01 M in PBS buffer, 0.1384 mmol[b.]). The mixture was stirred gently in a water bath at 37°C (internal temp.) for 3 hours under argon atmosphere to complete the reduction. The molar ratio (MR) of the thiol group to antibody was determined (as described in Example 5) initially and hourly thereafter (the MR should remain at about 14 to ensure complete reduction of the interchain S-S bonds).[c.] The relaxed (reduced) antibody was transferred to a 5 L reservoir connected to a Filtron Minisette fitted with an Omega tangential membrane[d.] in a cold room (4°C). The reduced antibody was diluted with 2 L PBS[b.] buffer (pH 7.4) and diafiltered/concentrated[e.] to about 350 ml. The solution was then transferred under argon atmosphere to a clean, sterile plastic container. The antibody concentration and molarity of the thiol group (and hence the MR of thiol group to antibody) was determined as above. A precooled solution of doxorubicin hydrazone (prepared as described in Example 3) (27.06 ml, 0.0063 M solution in water, 0.172 mmol, 1.1 equiv. to each thiol group[f]), was added to the relaxed antibody slowly with gentle stirring under a constant flow of argon in the cold room (4°C). 30 Minutes after the addition, the red conjugate solution was polish filtered through cellulose acetate filters (0.45μ followed by 0.22μ) and the resulting filtrate (350 ml) was divided into the following three portions: Portion 1 (150 ml), portion 2 (100 ml), and portion 3 (100 ml). Portion 1 (150 ml) was stirred with charcoal[g.] (0.75 grams) for 10 minutes in the cold room under argon atmosphere and filtered through a cellulose acetate membrane (0.22μ) to afford the title conjugate. The antibody concentration and the molarity of the doxorubicin (and hence the MR of antibody to doxorubicin) was then determined as described in Example 5. The antibody concentration was 8.18 mg/ml. The molar ratio was 7.96 and the pH was 7.22. The conjugate was frozen in 4 x 5 ml and 3 x 40 ml portions in sterile plastic bottles in liquid nitrogen and stored at -80°C.

Notes:

a. The pH of the BR 96 in citrate buffer (25 mM sodium citrate and 250 mM sodium chloride, pH = 5.5) was adjusted to 7.41 with saturated dibasic sodium phosphate (pH 9.0, about 10 ml).
b. PBS buffer was purchased from Gibco Laboratories Inc. and modified as follows: 500 ml of the PBS buffer (which contains 2.00 grams of potassium chloride, 2.00 grams of potassium phosphate monobasic, 80.00 grams of sodium chloride and 21.60 grams of sodium phosphate dibasic) was diluted with water for injection (WFI) to 5 L and the pH was adjusted to 7.4 with 1 N sodium hydroxide and the resulting solution was filtered through a 0.22 μ cellulose acetate membrane.
c. If the MR becomes low presumably due to the presence of trace metals in the reaction mixture, additional DTT should be added. Reduction can also be carried out in the presence of EDTA (1mM solution in PBS buffer) to chelate any oxidizing metals present in the antibody.
d. Minisette and Omega tangential membrane were purchased from Filtron. The membrane is made of polyethersulfone with 30,000 MW cut-off. The solution was pumped into the ultrafiltration system using Watson-Marlow 604 S/R perilstatic pump at 25 to 30 psi.
e. The rate of diafiltration/concentration was 65 ml/min. The effluent was monitored for MR of thiol to antibody. After about 1.6 L eluted, the MR of the effluent was 0.44. It was presumed that by this time most of the oxidized DTT and excess DTT was removed from the reaction mixture.
f. The volume of the required doxorubicin hydrazone was calculated according to the equation below.

$$\frac{\text{(Molarity of the thiol) x (volume of the relaxed antibody) x 1.1}}{6.3 \times 10^{-3}}$$

where $6.3 \times 10^{-3}$ is the molarity of the doxorubicin hydrazone solution and 1.1 is the number of equivalents of doxorubicin hydrazone to each thiol group.

g. The charcoal (Fisher Scientific, Cat # 7440-44-0, Norit, neutral) was washed while stirring with PBS buffer (pH 7.4) until the pH of the washings were constant at 7.4 and dried in vacuum.

Example 6

Optional Procedure for Preparation of Doxorubicin BR96 Conjugate (BR96 Monoclonal Antibody Conjugate to 6-Maleimidocaproylhydrazone of Doxorubicin)

Buffer Exchange From Citrate (pH 5.4) to Phosphate (pH 7.8) Buffer:

BR96 MAb, was supplied in citrate buffered saline[1] (CBS). Prior to the DTT reduction, BR96 in CBS buffer was diafiltered with five volumes of PBS buffer[2] using Filtron Minisette fitted with two cassettes.[3]

Preparation of Conjugate:

To the above solution of monoclonal antibody BR96 (480 ml, pH = 7.76, 0.032 m mole, conc. = 10.77 mg/ml in phosphate buffered saline[2]) in a 3-necked round bottomed flask was added, while stirring, a solution of dithiothreitol, DTT (16.2 ml, 0.01 M in PBS buffer, 0.162 m mole, 5.0 mole equiv.) in about 2 to 3 minutes. The mixture was stirred gently in a water bath at 37°C (internal temp.) for 4 hours under argon atmosphere to complete the reduction. The reaction mixture was then cooled in an ice bath. A precooled solution of doxorubicin hydrazone (prepared as described in Example 3, 56 ml, 0.0063 M solution in water, 0.356 m mole), was added to the reduced antibody slowly with stirring under a constant flow of argon at 0 - 4°C). 20 Minutes after the addition, the red conjugate solution was polish filtered through cellulose acetate membranes (0.45μ followed by 0.22μ). The resulting filtrate (510 ml) was stirred with charcoal[4] (7.0 grams) for 15 minutes at 0 - 4°C in the ice bath under argon atmosphere. It was filtered through two cellulose acetate membranes (0.45μ followed by 0.22μ) to afford the title conjugate (495 ml). The antibody concentration and the molarity of the doxorubicin (and hence the MR of antibody to doxorubicin) was then determined as described in Example 4. The antibody concentration was 8.42 mg/ml. The molar ratio was 8.26 and the pH was 7.44. The conjugate was then frozen as 4 x 100 ml, 5 x 5 ml and 2 x 35 ml portions in sterile plastic bottles in liquid nitrogen and stored at -80°C.

Notes:

1. BR96 in citrate buffer (25 mM sodium citrate and 250 mM sodium chloride, pH = 5.5).
2. PBS buffer , pH = 7.8 (0.01 M sodium phosphate and 0.1 M sodium chloride) was prepared as follows.

| | |
|---|---|
| $NaH_2PO_4 \cdot H_2O$ | 0.14 grams |
| $Na_2HPO_4$ | 1.28 grams |
| NaCl | 5.85 grams |
| Water (WFI) | QS to one liter |
| 1 N NaOH | ~0.7 ml (added till pH was 7.8) |
| Filtered through a 0.22μ cellulose acetate membrane. | |

3. Cassettes are membranes made of polyethersulfone with 30,000 MW cut-off.
4. The charcoal (Fisher Scientific, Cat # 7440-44-0, Norit, neutral) was washed while stirring with PBS buffer (pH 7.4) until the pH of the washings were constant at 7.4 and dried in vacuum.

Example 7

Purification of Doxorubicin-BR96 Conjugate with BIO-BEAD™

Crude conjugate (such as prepared in Example 4 or 5 without charcoal purification was purified by BIO-BEAD™ SM-2 in a batch process as follows:

The above crude conjugate (100 ml) and BIO-BEAD™ SM-2 (50.0 grams) were stirred in a sterile polystyrene flask in the cold room (4°C) under argon atmosphere for 30 minutes. The conjugate solution was filtered through a cellulose acetate filter (0.22μ) and MR (molar ratio of drug to antibody) was measured as described in Example 4 and found to be 8.12 and antibody concentration was 6.74 mg/ml. The conjugate was then frozen in 2 x 5 ml and 2 x 40 ml portions in sterile plastic bottles in liquid nitrogen and stored at -80°C.

1. The BIO-BEADS™ (Type: SM-2) made of polystyrene-divinylbenzene were purchased from Bio-Rad Laboratories. Prior to the use, they were washed successively with 0.5 N sodium hydroxide, water until neutral, methanol and water, and finally equilibrated (about 2 hours) with PBS buffer in a cold room.

Example 8

Purification of Doxorubicin-BR96 Conjugate by Dialysis

Crude conjugate such as prepared in Example 4 without charcoal purification (350 ml) was purified by diafiltration as follows:

The above conjugate was transferred into the reservoir of Amicon CH2RS Dialyzer fitted with a YM 30 spiral cartridge and diafiltered under argon atmosphere in the cold room. The flow rate of the filtrate was ~20 ml/minute. The conjugate was transferred to a sterile Nalgene PETE bottle. The antibody concentration and molarity of the doxorubicin ( and hence the MR of antibody to doxorubicin) was then determined. The pH of the conjugate was 7.6. The antibody concentration was 9.2 mg/ml and the molar ratio was 8.25. The product was frozen as 3 x 100 ml, 1 x 20 ml and 2 x 5 ml portions in sterile plastic bottles in liquid nitrogen and stored at -80° C.

Example 9

Purification of Doxorubicin-BR96 Conjugate by Cation Exchange

A. Resin preparation, column packing and equilibration:

CM SEPHADEX™ C-25 (500 g) was allowed to swell at room temperature in 0.5 N NaCl (2 L) for 2 h. After decanting the supernatant aqueous layer the wet resin was stored in the PBS buffer[2] (4 L) at room temperature for 2 days. The PBS buffer was decanted and replaced with 1 N NaCl (3 L) and the swollen resin[3] was used for packing the column after 16 hours.

The assembled BPG 100 (Pharmacia Inc.) column[4] was initially rinsed with 1 N NaCl. The swollen CM Sephadex C-25 resin in 1 N NaCl (total vol 3 L) was slowly poured into the column and packed. The column was then equilibrated by eluting/washing with the PBS buffer (3 vols). [5] The testing of the column was done using acetone and 1N NaCl. [6] The packed column was stored at ~4° for 2 days to equilibrate before use.

B. Congugate purification:

Freshly prepared crude conjugate[7] (such as prepared in Example 4 without charcoal purification) conc.[8] = 10.5 mg/mL and MR = 10.0; 430 mL) in PBS buffer was loaded[9] on the above column at 4 °C in about 12 minutes at a volumetric flow rate of 35 ml/min using a peristaltic pump. The conjugate was eluted with the PBS buffer at about 60 mL flow rate. The eluent was collected into three fractions in about 9 minutes; initially a 40 ml fraction, followed by the major portion of the conjugate in 465 ml as a single fraction and a very dilute third fraction of 65 mL at the end.

| Results from the UV analysis of the fractions: | | | |
|---|---|---|---|
| Fractions | I | II | III |
| Vol. collected (mL) | 40 | 465 | 65 |
| Protein (gm) | 0.1 | 4.36 | 0.09 |
| MR | 6.84 | 7.64 | 8.16 |
| %Recovery | 2.0 | 96.0 | 2.0 |

C. Column regeneration and equilibration:

After the conjugate was eluted the column was regenerated by washing with 1 N NaCl (3 vols) followed by washing/eluting with the PBS buffer (3 vols).[10]

Notes:

1. CM SEPHADEX™ C-25 is a modified Sephadex G-25 weak cation exchange resin with an exclusion limit = $3 \times 10^4$, bead diameter = 40 - 125 µm and the total ionic capacity = 4 - 5 µmol/mL.
2. PBS buffer (Phosphate buffered saline, pH = 7.5): $NaH_2PO_4 \cdot H_2O$ 0.00117 M; $Na_2HPO_4$ 0.009 M; NaCl 0.1 M; ionic strength 0.1282; conductivity 9.39 mS.
3. 500 g of the resin was swollen to 2800 ml.
4. Column dimensions: L = 36 cm; D = 10 cm: bed vol = ~ 2800 ml.
5. The column was packed and equilibrated at room temperature and transferred to the cold room at 4°C. The column was allowed to equilibrate at this temperature for 16 hours before loading the conjugate.
Flow rate (28 psi): [Volumetric] = 215 mL/min; [Linear] = 2.74 mL/min/cm$^2$.
6. HETP in this case was 0.072. A value between 0.1 and 0.01 is an acceptable number (Pharmacia). HETP (height equivalent to a theoretical plate) = L/N, where, L = bed height (cm) and N = no. of theoretical plates. $N = 5.54(V_e/W_{1/2})^2$, where, $V_e$ = Elution vol (mL) and $W_{1/2}$ = peak width at half height (mL)
7. The crude conjugate was polish filtered through cellulose acetate membranes (prefilter 0.44µ followed by 0.22µ) before charging on the column.
8.The antibody molar concentration (mg/mL) was determined using PE Lambda 6 UV/visible spectrophotometer.

$$MC_{antibody} = \frac{A_{280} - (0.724 \cdot A_{495})}{1.4}$$

where 0.724 is the correction factor for doxorubicin absorbance at 280 nm and 1.4 is the UV absorbance at 280 nm for the antibody.
9. Material loading ratio (bed vol:vol of the conjugate) = 6.5:1
10. Column regeneration and equilibration took about 1.5-2 hours.

However, after using the column for 3 times, a distinct red thin layer was observed at the top of the resin. Column regeneration method did not completely remove the red color.

UV absorbance of the eluent after regeneration and equilibration showed zero readings at 280 nm and 495 nm.

Example 10

Purification of Doxorubicin-BR96 Conjugate by Column Chromatography

Column preparation:

The preparation of CM Sephadex C-25 resin (as described in Example 9), column packing, initial washing and regeneration have been described in Example 9.

Conjugate purification:

Freshly prepared crude conjugate (such as prepared in Example 4 without charcoal purification) (conc. = 9.01 mg/mL and MR = 12.31; 430 mL) in PBS buffer (as in Example 9) was loaded on the above column (as described in Example 9) (which was equilibrated for 16 hours at 4°C) in about 9 minutes at a volumetric flow rate of 50 ml/min using a peristaltic pump. The conjugate was eluted with the PBS buffer at about 60 mL/minute flow rate. The eluent was collected into three fractions in about 9 minutes; initially a 40 ml fraction, followed by the major portion of the conjugate in 450 ml fraction and a very dilute third fraction of 60 mL at the end.

| Results from the UV analysis of the fractions: | | | |
|---|---|---|---|
| Fractions | I | II | III |
| Vol. collected (mL) | 40 | 450 | 60 |
| Protein (gm) | 0.08 | 3.66 | 0.08 |
| MR | 7.7 | 8.42 | 8.41 |
| %Recovery | 2.0 | 95.0 | 2.0 |

Column regeneration and equilibration:

After the conjugate was eluted the column was regenerated (as described in Example 9) by washing with 1 N NaCl (3 vols) followed by washing/eluting with the PBS buffer (3 vols).

Example 11

Concentration of Doxorubicin-BR96 Conjugate by Diafiltration

The purified conjugate (3520 ml; concentration is 8.5 mg/ml) such as prepared in Example 4 was concentrated as follows:

The above conjugate was transferred into the reservoir on Amicon CH2RS Dialyzer fitted with a YM 30 regenerated cellulose acetate spiral cartridge and concentrated under argon atmosphere in the cold room. The flow rate was ~20m./min. The conjugate was concentrated to about 1785 ml (concentration is 17.3 mg/ml) and transferred to a sterile Nalgene PETE bottle. The antibody concentration and molarity of the doxorubicin (and hence the MR of antibody to doxorubicin) was determined. The pH of the conjugate was 7.7. The antibody concentration was 17.3 mg/ml and the molar ratio was 8.39. The product was frozen as 17 x 100 ml, 1 x 55 ml, and 6 x 5 ml portions in sterile plastic bottles in liquid nitrogen and stored at ~80°C.

## Claims

1. A method for preparing a thioether conjugate of the structure

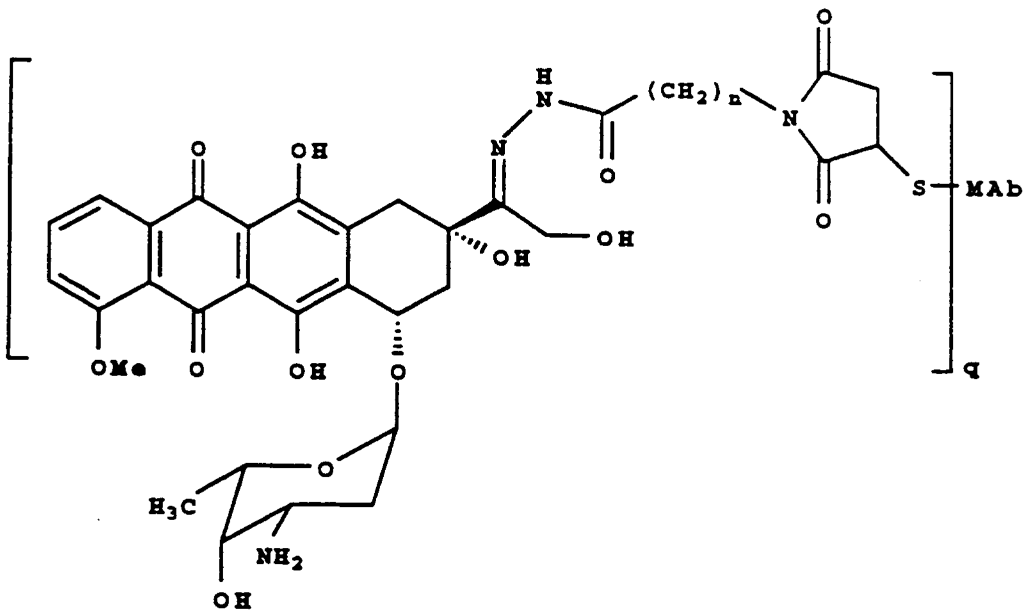

wherein n is an integer of from about 1 to about 10;

q is from 4 to 10;

MAb is an immunoglobulin which is a relaxed chimeric BR96 antibody, a BR96 antibody, a chimeric BR96 antibody or a relaxed BR96 antibody, or an antigen-binding fragment thereof, which comprises in a first method

(a) providing a buffered solution of reduced form of immunoglobulin thioether of the structure

$MAb\text{-}(SH)_{6-9}$

containing a phosphate buffer having a pH of within the range of from about 6 to about 9;

(b) diafiltering the buffered solution of reduced immunoglobulin thioether under an inert atmosphere to provide a diafiltered solution;

(c) reacting the diafiltered solution of the immunoglobulin thioether with a solution of doxorubicin hydrazone of the structure

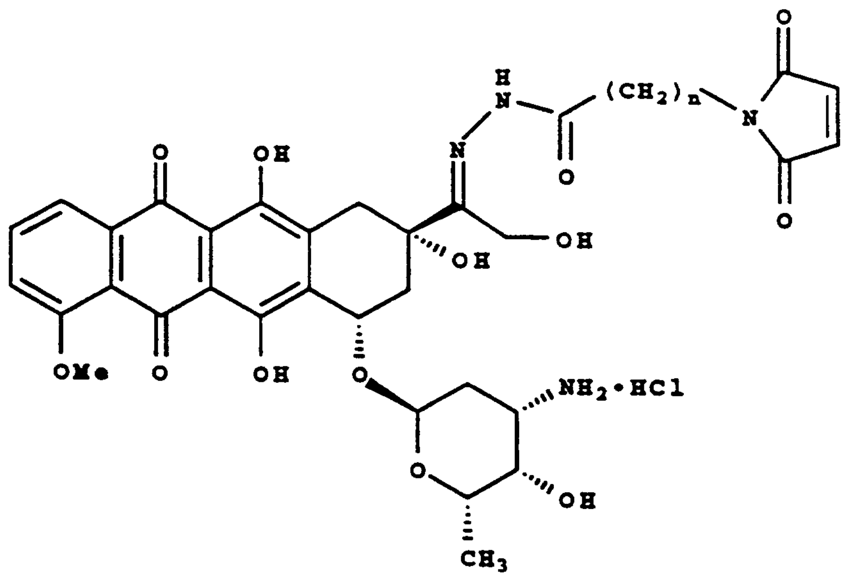

wherein n is as defined above,
under an inert atmosphere to form crude thioether conjugate; and
(d) purifying the thioether conjugate; or which comprises in a second method
(a) providing a diafiltered phosphate buffered solution of immunoglobulin thioether of the structure

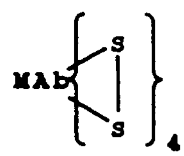

said solution having a pH of within the range of from about 6 to about 9;
(b) treating the phosphate buffered solution of immunoglobulin thioether with a phosphate buffered solution of reducing agent under an inert atmosphere to form a buffered solution of reduced immunoglobulin thioether of the structure

MAb-$(SH)_{6-9}$ ;

(c) reacting the buffered solution of reduced immunoglobulin thioether with a solution of doxorubicin hydrazone of the structure

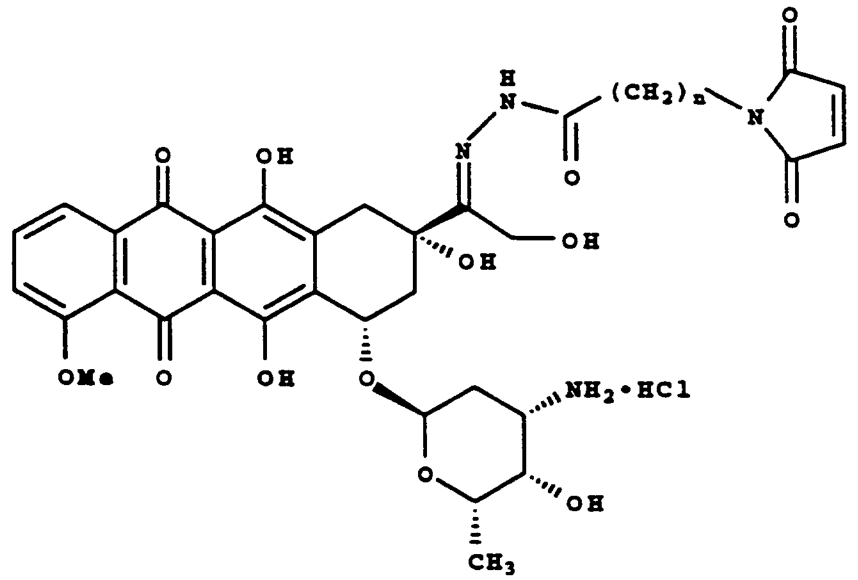

,

wherein n is as defined above,
under an inert atmosphere, to form a crude thioether conjugate; and
(d) purifying the thioether conjugate.

2. The method as defined in Claim 1 wherein in the first method the buffered solution of reduced form of immunoglobulin thioether is prepared by
(a) providing an immunoglobulin thioether of the structure

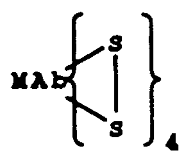

in citrate buffer, said citrate buffer having a pH of within the range of from about 4 to about 6;
(b) treating the buffered immunoglobulin thioether with a phosphate buffered solution having a pH of within the range of from about 6 to about 10, to provide a buffered solution of immunoglobulin thioether having a pH of within the range of from about 6 to about 9; and
(c) treating the buffered immunoglobulin thioether with a phosphate buffered solution of reducing agent, under an inert atmosphere, to form a buffered solution of reduced immunoglobulin thioether.

3. The method as defined in Claim 1 wherein n is 5 and MAb is relaxed chimeric BR96 antibody.

4. The method as defined in Claim 2 wherein the reducing agent is dithiothreitol.

5. The method as defined in Claim 2 wherein the reaction of the buffered immunoglobulin thioether and buffered reducing agent is carried out at a temperature within the range of from about 25 to about 45 °C, under an argon atmosphere, for a period of from about 1 to about 5 hours.

6. The method as defined in Claim 1 wherein diafiltering of the buffered solution of reduced immunoglobulin thioether is carried out at a temperature within the range of from about -5 to about 25 °C, under an argon atmosphere.

7. The method as defined in Claim 1 wherein the reaction of the diafiltered solution of the immunoglobulin thioether and the doxorubicin hydrazone is carried out at a temperature within the range of from about

-5 to about 25 ° C, under an argon atmosphere, for a period of from about 10 to about 60 minutes.

**8.** The method as defined in Claim 2 wherein the reducing agent is employed in a molar ratio to immunoglobulin thioether of within the range of from about 10:1 to about 5:1.

**9.** The method as defined in Claim 1 wherein the molar ratio of reduced immunoglobulin thioether to doxorubicin hydrazone is within the range of from about 1:4 to about 1:11.

**10.** The method as defined in Claim 1 wherein the doxorubicin hydrazone is employed in an amount to provide from about 1.0 to about 1.5 equivalents for each thiol of the reduced immunoglobulin thioether.

**11.** The method as defined in Claim 2 wherein the citrate buffered saline is comprised of from about 10 mM to about 75 mM sodium citrate and about 50 mM to about 500 mM sodium chloride and pH from about 5 to about 6.5.

**12.** The method as defined in Claim 2 wherein the phosphate buffer is an alkali metal phosphate buffer.

**13.** The method as defined in Claim 2 wherein the phosphate buffered saline is comprised of about 0.005 M to about 0.02 M sodium phosphate and about 0.05 M to 0.2 M sodium chloride and pH about 7-8.

**14.** The method as defined in Claim 2 wherein the phosphate buffered saline is comprised of
from about 0.04 to about 0.4% by weight KCl
from about 0.4 to about 4% by weight $Na_2HPO_4$
from about 0.04 to about 0.4% by weight $KH_2PO_4 \cdot H_2O$.

**15.** The method as defined in Claim 1 wherein the crude thioether conjugate is purified by filtering the crude conjugate and treating the filtered conjugate with carbon or charcoal.

**16.** The method as defined in Claim 1 wherein the crude thioether conjugate is purified by membrane filtration.

**17.** The method as defined in Claim 16 wherein the crude thioether conjugate is purified by passing it through regenerated cellulose acetate, polyethersulfone, polysulfone or hydrophilized polyacrylonitrile.

**18.** The method as defined in Claim 1 wherein the crude thioether conjugate is purified by use of beads.

**19.** The method as defined in Claim 18, wherein the Bio-Beads are used in a batch process.

**20.** The method as defined in Claim 1 wherein the crude thioether conjugate is purified by passing the conjugate through a chromatographic column containing a gel or resin filtration medium, or by passing the conjugate through an ion-exchange column.

**21.** The method as defined in Claim 20 wherein the crude thioether conjugate is purified by employing gel filtration or is purified by ion-exchange
or is purified by hydrophobic interaction.

**22.** The method as defined in Claim 1 wherein in the second method diafiltered phosphate buffered solution of immunoglobulin thioether of the structure

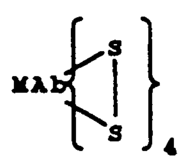

is prepared by

(a) providing a solution of immunoglobulin thioether in citrate buffered saline, said citrate buffered saline having a pH of within the range of from about 4 to about 6; and

(b) diafiltering said citrate buffered immunoglobulin thioether with phosphate buffer, said phosphate buffer having a pH within the range of from about 6 to about 9, to provide a diafiltered phosphate buffered solution of immunoglobulin thioether having a pH within the range of from about 6 to about 9.

**23.** The method as defined in Claim 22 wherein in the second method the solution of the citrate buffered immunoglobulin thioether is concentrated to from about 10 mg/ml to about 30 mg/ml immunoglobulin thioether prior to the diafiltering step.

**24.** The method as defined in Claim 1 wherein the phosphate buffered solution of the immunoglobulin thioether is treated with a chelating agent to remove metallic impurities present in the immunoglobulin thioether, phosphate buffering agent or both.

**25.** A method for preparing a hydrazide acid salt compound of the structure

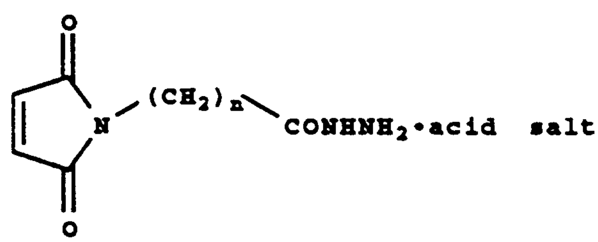

wherein n is 1 to 10, which comprises

(a) subjecting a crystallized form of cyclized acid of the structure

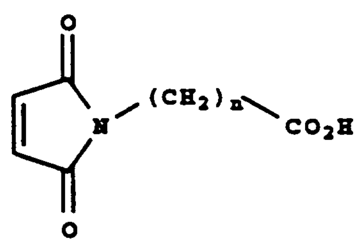

to a coupling reaction by treating the cyclized acid with a coupling reagent including an alkylcarbazate to form a carbazate of the structure

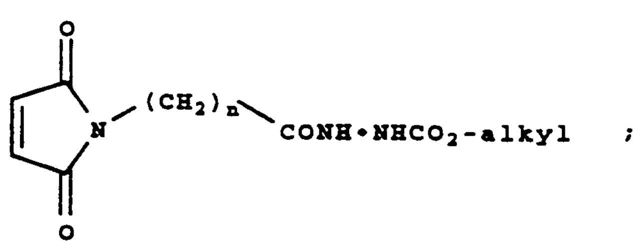

and

(b) deprotecting the above carbazate intermediate by reacting same with an acid to form the hydrazide acid salt.

**26.** The method as defined in Claim 25 wherein the starting cyclized acid is prepared by

(a) reacting maleic anhydride with an amino acid of the structure

$H_2N-(CH_2)_n-COOH$

in the presence of a weak organic acid to form an acid condensation product of the structure

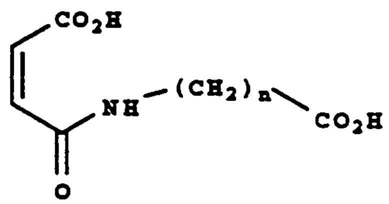

;

and

(b) treating the above acid with a cyclizing agent in the presence of a weak organic base and an inert organic solvent, to form the cyclized acid of the structure

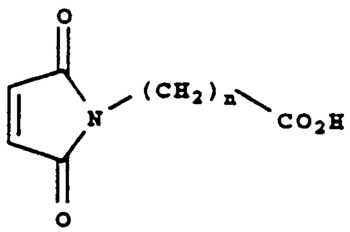

**27.** The method as defined in Claim 25 wherein the starting cyclized acid is prepared by reacting maleic anhydride with an amino acid of the structure

$H_2N-(CH_2)_n-COOH$

in the presence of an organic acid under an inert atmosphere.

**28.** The method as defined in claim 26 wherein the maleic anhydride is reacted with an amino acid which is aminocaproic acid.

**29.** The method as defined in Claim 28 wherein the reaction of the maleic anhydride and aminocaproic acid is carried out in the presence of acetic acid.

**30.** The method as defined in Claim 29 wherein the cyclizing agent employed in cyclizing the acid condensation product is chlorotrimethyl silane or hexamethyldisilazane, the weak organic base is triethylamine or diisopropylethylamine and the inert organic solvent is acetonitrile, to form maleimidocaproic acid.

**31.** The method as defined in Claim 30 wherein the maleimidocaproic acid is crystallized employing ethyl acetate and hexane.

**32.** The method as defined in Claim 30 wherein the cyclization is carried out at a temperature within the range of from about 70 to about 100 °C.

**33.** The method as defined in Claim 25 wherein the coupling reagent employed to effect coupling of the cyclized acid includes t-butylcarbazate, N-methylmorpholine and isobutylchoroformate and an inert organic solvent to form the carbazate intermediate.

**34.** The method as defined in Claim 33 wherein the carbazate intermediate is isolated by crystallization with ethyl acetate and hexane.

**35.** The method as defined in Claim 25 wherein the carbazate intermediate is deprotected by reacting same with trifluoroacetic acid to form the corresponding trifluoroacetic acid salt or by reacting same with hydrochloric acid to form the corresponding hydrochloride salt.

**36.** The method for forming a t-butylcarbazate intermediate of the structure

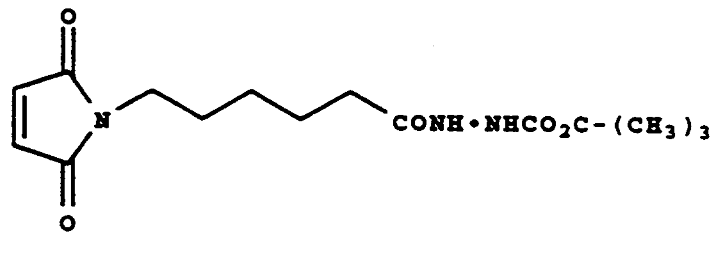

which comprises subjecting maleimidocaproic acid to a coupling reacting by treating said acid with N-methyl morpholine, isobutylchloroformate and an inert organic solvent and then with t-butylcarbazate in the presence of an inert organic solvent.

**37.** A process for concentrating the purified doxorubicin-BR96 conjugate as defined in Claim 1 to a desired concentration of from about 10 mg/ml to about 50 mg/ml by employing a diafiltration technique.

**38.** A process for preparing the doxorubicin hydrazone as defined in Claim 1 which comprises reacting doxorubicin hydrochloride with a hydrazide acid salt of the structure

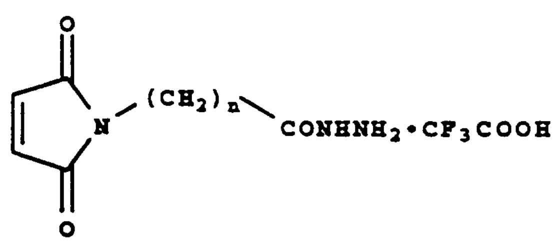

in the presence of acid, and an alcohol solvent under an inert atmosphere to form doxorubicin hydrazone, and isolating the doxorubicin hydrazone.

**39.** The process as defined in Claim 38 wherein the doxorubicin hydrazone is isolated by reaction of the doxorubicin hydrazone with ethyl acetate, acetonitrile or tetrahydrofuran at a reduced temperature and filtering to collect the doxorubicin hydrazone and optionally washing the hydrazone with cold solvent.